# EUROPEAN PATENT APPLICATION

(11) **EP 2 399 938 A2**
(43) Date of publication of application: **28.12.2011**
(21) Application number: 10182124.7
(22) Date of filing: 14.06.2005
(51) Int. Cl.: C07K 17/10, C07K 1/22, B01J 20/26, B01J 45/00, C07K 7/08, C07K 7/06, C12P 21/02, C12N 15/63

(54) **Peptide purification by means of hard metal ion affinity chromatography**

(30) Priority: 14.06.2004 DK 200400916
(62) Divisional of application: 05760981.0
(71) Applicant: Novo Nordisk A/S, 2880 Bagsværd (DK); Monash University, Clayton VIC 3168 (AU)
(72) Inventor: Hearn, Thomas William Milton, Balwyn, Victoria 3103 (AU); Spiccia, Leone, Wheelers Hill, Victoria 3150 (AU); Daly, Rachel, Bonbeach, Victoria 3196 (AU); Krehrer, Ute, Cheltenham, Victoria 3192 (AU)
(74) Representative: Søndergård, Mette Weigelt

(57) **Abstract**

A polymer substrate functionalized with a functionality comprising at least one cyclic, metal ion coordinating ligand group which comprises at least 3 nitrogen donor atoms in the ring of the cyclic group, at least one of the nitrogen atoms having an optionally substituted carboxy(lower alkyl) or optionally substituted phosphono(lower alkyl) group covalently attached thereto, is well suited for use in conjunction with "hard" metal ions of low toxicity (such as Ca²⁺, Mg²⁺ or Fe³⁺) in the separation/purification of appropriately "tagged" polypeptides by Immobilized Metal ion Affinity Chromatography (I MAC).

SEQUENCE LISTING

<110>  Novo Nordisk A/S and Monash Univeristy

<120>  PEPTIDE PURIFICATION BY MEANS OF HARD METAL ION AFFINITY
       CHROMATOGRAPHY

<130>  6935

<160>  8

<170>  PatentIn version 3.3

<210>  1
<211>  14
<212>  PRT
<213>  ARTIFICIAL

<220>
<223>  purification tag

<400>  1

Met Asp Ala Asp Gly Asn Gly Thr Ile Asp Phe Ala Glu Phe
1               5                   10


<210>  2
<211>  14
<212>  PRT
<213>  ARTIFICIAL

<220>
<223>  purification tag

<400>  2

Met Asp Ile Asp Gly Asp Gly His Ile Asn Tyr Glu Glu Phe
1               5                   10


<210>  3
<211>  14
<212>  PRT
<213>  ARTIFICIAL

<220>
<223>  purification tag

<400>  3

Met Asp Val Asp Gly Ser Gly Thr Ile Gly Ser Ser Glu Leu
1               5                   10


<210>  4
<211>  14

<212>  PRT
<213>  ARTIFICIAL

<220>
<223>  purification tag

<400>  4

Met Asp Val Asp Arg Ser Gly Thr Ile Gly Ser Ser Glu Leu
1               5                   10


<210>  5
<211>  6
<212>  PRT
<213>  ARTIFICIAL

<220>
<223>  purification tag

<400>  5

Met Asp Ala Asp Gly Asn
1               5


<210>  6
<211>  6
<212>  PRT
<213>  ARTIFICIAL

<220>
<223>  purification tag

<400>  6

Met Asp Ile Asp Gly Asp
1               5


<210>  7
<211>  6
<212>  PRT
<213>  ARTIFICIAL

<220>
<223>  purification tag

<400>  7

Met Asp Val Asp Gly Ser
1               5


<210>  8
<211>  6
<212>  PRT

<213>  ARTIFICIAL

<220>
<223>  purification tag

<400>  8

Met Asp Val Asp Arg Ser
1               5

## Description

### FIELD OF THE INVENTION

The present invention relates, *inter alia*, to the field of isolation and purification of peptides, notably polypeptides, such as recombinant proteins, by means of immobilized hard metal ion affinity chromatography.

### BACKGROUND OF THE INVENTION

An important aspect of the production of recombinant (genetically engineered) peptides, including oligo- and polypeptides, notably proteins, intended for therapeutic use in humans or animals is purification of the peptides in question to a sufficiently high level of purity, such that the desired protein is essentially completely free of contamination with, in particular, (a) any extraneous proteins which may arise in the production process (typically a fermentation process or the like employing a selected or genetically modified strain of an appropriate microorganism) and (b) undesirable metal ions (notably heavy-metal ions) that may have been introduced in the course of the production process.

Immobilized metal ion affinity chromatography (IMAC) is a versatile separation procedure that exploits differences in the affinities exhibited by many biopolymers for metal ions. The technique involves the chelation of a suitable metal ion onto a solid support matrix whose surface has previously been chemically modified with a polydentate ligand. The resulting immobilized metal ion chelating complex then has the potential to coordinate with one or more electron donor groups resident on the surface of the interacting protein (Sulkowski, E., Trends in Biotechnology, 3 (1985) 1-6; Porath, J., Carlsson, I., Olsson, I. and Belfrage, G., Nature, 258 (1975) 598-599; Kagedal, L., in "Protein Purification" (Ed., J. C. Janson, and L. Ryden), VCH Publishers (1989) pp. 227-251; Zachariou, M. and Hearn, M. T. W., Biochemistry, 35 (1996) 202-211. Separation selectivity is then achieved on the basis of differences in the thermodynamic stabilities of the immobilized metal ion complexes with the various adsorbed proteins. Proteins whose adsorption complexes are the least stable will be eluted first, whilst proteins that form more stable complexes will be eluted later. The greater the difference in the equilibrium association constants, i.e. the larger the differences in the dissociation constants (K_{D}) of the respective protein/immobilized metal ion coordination complexes, the higher the resolution obtained. Consequently, the amino acid composition, surface distribution of particular amino acid residues, as well as the conformation of the protein all play important roles in determining the affinity of a protein for a particular IMAC system. As a result, proteins with very similar properties with respect to charge, molecular size and amino acid composition, but with differences in their tertiary structures, may be resolved.

Most of the research interest into the use of IMAC over the past 20 years has revolved around the application of 1st row transition metal ions of borderline hardness (*vide infra*), such as Cu²⁺, Zn²⁺ and Ni²⁺. These metal ions demonstrate intermediate metal ion stability constants, e.g. logβ values between 5 and 10, for both aromatic and aliphatic amines, as well as for carboxylate functional groups (Wong, J. W., Albright, R. L. and Wang, N. H. L., Separation and Purification Methods, 20 (1991) 49-57; Zachariou, M., Traverso, I.., Spiccia, L. and Hearn, M.T.W., Journal of Physical Chemistry, 100 (1996) 12680-12690). A number of unconstrained tridentate chelates that exhibit these binding properties with M²⁺ ions can be chemically immobilized onto support materials. Despite their limitations with regard to the magnitude of the corresponding logβ values and their resulting relatively low selectivity capabilities, unconstrained types of chelating compounds such as iminodiacetic acid (IDA) constitute the principal types of chelating ligand employed hitherto in such IMAC investigations [see, e.g., Kagedal, L., in "Protein Purification" (Eds. J. C. Janson and L. Ryden), VCH Publishers (1989) pp 227-251]. Applications illustrative of the use of immobilized M²⁺-IDA-based IMAC systems include the purification of α-amylases from germinated wheat using immobilized Cu²⁺-IDA [Zawistowska, U., Sangster, K., Zawistowski, J., Langstaff, J. and Friessen, A. D., Cereal Chemistry, 65 (1988) 5413-5418]; and purification of human clotting factor VII [Weeransinghe, K. M., Scully, M. F. and Kadder, V. V., Biochimica Biophysica Acta, 839 (1985) 57-65] and of α₁-thiol proteinases [Otsuka, S. and Yamanaka, T. (Eds), "Metalloproteins -Chemical Properties and Biological Effects" in "Bioactive Molecules", Kodansha Ltd, Tokyo (1988), pp 18-45] from human plasma using immobilized Zn²⁺-IDA. An extension of the use of IDA-based IMAC procedures, viz. the purification of recombinant proteins using immobilized Ni²⁺-nitrilotriacetic acid (Ni²⁺-NTA) [Hochuli, E., Bannwarth, W., Döbeli, H. and Stuber, D., Bio/Technology, 6 (1988) 1321-1324] (NTA being a structural homologue of IDA), relies on the incorporation at the gene level of a polynucleotide sequence corresponding to a poly-histidine peptide, typically hexa-His, which confers on the protein a higher affinity for binding to immobilized Ni²⁺-NTA chelating complex, thus enabling the protein to be selectively retained on this IMAC sorbent. In the present application, the terms "sorbent" and "adsorbent" are used primarily to denote a functionalized polymer substrate (polymer substrate with ligand immobilized thereto) with coordinatively bound metal ion(s), although these terms are also occasionally employed to denote a functionalised polymer substrate without metal ion(s) bound thereto.

As will be noted from the above description of applications of IDA- and NTA-based IMAC systems, an alternative means of altering protein binding selectivity with IMAC systems is through variation in the structure of the chelating ligate. In recent years however, only a handful of new IMAC chelating ligates have been introduced. These include systems based on the bidentate chelators aminohydroxamic acid (AHM) and 8-hydroxyquinoline (8-HQ) [Zachariou, M., Traverso, I., Spiccia, L. and Hearn, M.T.W., Journal of Physical Chemistry, 100 (1996) 12680-12690]; carboxymethylaspartic acid (CM-ASP) which has a higher affinity for Ca²⁺ than IDA [Porath, J., Trends in Analytical Chemistry, 7 (1988), 254-256; Mantovaara, T., Pertofz, H. and Porath, J., Biotechnology Applied Biochemistry, 11 (1989), 564-569]; ortho-phosphoserine (OPS), which is able to chelate "hard" metal ions such as Fe³⁺, Al³⁺, Ca²⁺ and Yb³⁺ due to the participation of the phosphate group [Zachariou, M., Traverso, I. and Hearn, M. T. W., Journal of Chromatography, 646 (1993), 107-115]; and other tridentate ligates, such as (2-pyridylmethyl)aminoacetate (CPMA), dipicolylamine (DPA) and cis- or trans-carboxymethyl-proline [Chaouk, H., Middleton S., Jackson W.R. and Hearn, M.T.W., International Journal of BioChromatography, 2 (1997) 153-190; Chaouk, H. and Hearn, M.T.W., Journal of Biochemical and Biophysical Research Methods, 39 (1999) 161-177], tetradentate ligands, such as nitrilotriacetic acid (NTA) [Hochuli, E., Bannwarth, W., Dobeli, H., Gentz, R. and Stuber, D. Bio/Technology, 6 (1988) 1321-1325], which have higher affinities for M²⁺ ions than IDA due to their quadridentate nature, exhibit lower protein binding association constants due to the loss of one coordination site compared to the IDA-type tridentate ligates; and pentadentate ligands, such as tetraethylenepentamine (TEPA) [Hidaka Y., Park, H. and Inouye, M., FEBS Letters, 400 (1997) 238-242] or N,N,N'-tris(carboxymethyl)ethylene-diamine (TED) [Porath, J., Protein Expression & Purification, 3 (1992) 263-281], which coordinate metal ions via five donor atoms (i.e. two nitrogen atoms of primary amine groups and three nitrogen atoms of secondary amine groups in the case of TEPA, and two nitrogen atoms of secondary amine groups and three oxygen atoms from the three carboxylic groups in the case of TED).

Significant leakage of metal ions has been observed with immobilized metal ion iminodiacetic acid chelate (im-Mⁿ⁺-IDA) systems when using relatively mild elution conditions in the chromatographic process [Oswald, T., Hornbostel, G., Rinas, U. and Anspach, F.B., Biotechnology Applied Biochemistry, 25 (1997) 109-115; Kagedal, L. in Protein Purification (eds. J.C. Janson and L Ryden) VCH Publishers, New York (1989), pp 227-251]. Thus, in addition to the issue of selectivity modulation, an additional motivation for the development of new classes of chelating ligates has been a need for achieving significant increases in the metal ion stability constants compared to the IDA-based or NTA-based systems which have hitherto been employed [Zachariou, M., Traverso, I., Spiccia, L. and Hearn, M.T.W., Analytical Chemistry, 69 (1996) 813-822].

WO 03/042249 relates, *inter alia*, to classes of functionalized polymer substrates containing a functionality comprising one or more cyclic, metal ion coordinating ligand groups having at least 3 metal ion coordinating donor atoms chosen independently among N, O and S. When employed as a matrix for one or more metal ions that form(s) coordination bonds to these donor atoms whilst retaining vacant coordination sites, these functionalized polymer substrates were found to exhibit remarkably high strength and/or selectivity of binding towards fusion proteins in the form of proteins or polypeptides "tagged" with an additional oligopeptide sequence ("tag") incorporating one or more appropriately positioned amino acid residues capable of forming a coordination bond to the vacant coordination site(s) of the metal ion or ions in question. The generally significantly greater strength and/or selectivity of binding of the fusion protein to such a matrix compared with that of the binding of extraneous proteins then facilitated separation and isolation of the fusion protein from a mixture containing the fusion protein together with one or more extraneous proteins. Preferred functionalized (metal ion coordinating) polymer substrates disclosed in WO 03/042249 employ functionalities in which the metal ion coordinating donor atoms in each cyclic, metal ion coordinating group consist of three nitrogen donor atoms in the ring, and they are particularly well suited for use as a matrix for certain metal ions of borderline properties with respect to "hardness" or "softness" (*vide infra*), such as Cu²⁺ or Ni²⁺ . Functionalized polymer substrate systems well suited as a matrix for "hard" metal ions (such as Ca²⁺, Mg²⁺, Mn²⁺ and Fe³⁺) or metal ions at the "hard" end of the scale with respect to "borderline" hardness (such as Zn²⁺) are less well represented in WO 03/042249.

### BRIEF DESCRIPTION OF THE INVENTION

One objective of the present invention was to provide novel IMAC systems based on "hard" metal ions (such as Ca²⁺, Mg²⁺ and Fe³⁺) and metal ions at the "hard" end of the scale with respect to "borderline" hardness (such as Zn²⁺) that function through interactions with hard donor atoms [especially oxygen atoms in carboxylate groups (as in Asp or Glu amino acid residues) and/or phosphate groups] present in biomolecules. An important feature of these novel IMAC-based systems is that they can concurrently achieve a mixed modality of interaction with their target molecules that is based on a combination of coordination (electron donor/electron acceptor) and electrostatic (ion-exchange) processes. As a consequence, these novel IMAC-based systems can function with selectivity mediated through mixed modes of interaction that are unique and thus offer the opportunity for a new capability in protein purification.

One aspect of the present invention thus relates to a polymer substrate functionalized with a functionality comprising at least one cyclic, metal ion coordinating ligand group which comprises at least 3 nitrogen donor atoms in the ring of said cyclic group, at least one of said nitrogen atoms having an optionally substituted carboxy(lower alkyl) group or an optionally substituted phosphono(lower alkyl) group covalently attached thereto. A second aspect of the invention relates to a functionalized polymer substrate of the latter type, further comprising a metal ion coordinated to at least one of the cyclic ligand groups in the functionality. Other aspects of the invention include methods for preparing such functionalized polymer substrates.

Further important aspects of the present invention relate to:
oligopeptides that are well suited for incorporation as "tags" in fusion proteins in the context of the present invention;
fusion proteins of the type in question, comprising a protein of interest fused at its amino terminus or carboxy terminus or both, or alternatively at a location within the internal amino acid sequence of the protein of interest, to at least one such oligopeptide;
polynucleotide constructs, e.g. vectors, encoding such fusion proteins;
host cells that comprise such a polynucleotide construct;
a method for producing a fusion protein of the type in question, wherein a host cell of the latter type is cultivated in a growth medium under conditions whereby the fusion protein is expressed, and whereby the fusion protein is recovered from the medium; and
a method for purifying a protein of interest, wherein a wild-type protein or a protein sample containing such a fusion protein (comprising the protein of interest) as well as other proteins (extraneous proteins) is contacted with a functionalized polymer substrate according to the invention or a metal ion-containing functionalized polymer substrate according to the invention.

### DETAILED DESCRIPTION OF THE INVENTION

As already indicated above, a first aspect of the invention relates to a polymer substrate functionalized with a functionality comprising at least one cyclic, metal ion coordinating ligand group which comprises at least 3 nitrogen donor atoms in the ring of the cyclic group, at least one of the nitrogen atoms having an optionally substituted carboxy(lower alkyl) or optionally substituted phosphono(lower alkyl) group covalently attached thereto.

Useful polymer substrates in the context of the invention include both water-soluble polymers and substantially water-insoluble polymers, and may be selected from a very wide range of polymeric materials. Examples hereof are the following:
Polysaccharides and derivatives thereof, including agaroses, dextrans, celluloses, hemicelluloses, starches, xylans and the like, and derivatives of these polysaccharides. Suitable polysaccharide derivatives will, in general, include derivatives in which some proportion of the hydroxy groups of the polysaccharide in question is derivatized to form ethers (e.g. lower alkyl ethers, such as methyl ethers) or esters (e.g. lower carboxylic acid esters, such as acetate, propionate and the like), as well as materials in which the starting polysaccharide or a derivative thereof has been cross-linked by treatment with an appropriate cross-linking reagent.

Generally speaking, functionalized polymer substrates of the invention based on substantially water-insoluble polymers are, for example, well suited for packing into chromatography columns, for direct introduction into a medium (batchwise use) and the like, and polysaccharides that are particularly well suited for this type of application in the context of the invention include agaroses, dextrans and derivatives thereof, a variety of suitable types of which are readily commercially available. Thus, for example, a variety of agarose products are produced by Amersham Pharmacia Biotech, Uppsala, Sweden, and marketed under the name Sepharose™; available grades include Sepharose™ 2B, 4B and 6B. Cross-linked derivatives of these various grades of agarose (prepared by cross linking of Sepharose™ with 2,3-dibromopropanol) are also available from the same company, and are marketed as Sepharose™ CL-2B, CL-4B and CL-6B, Sepharose™ 4 and 6 Fast Flow, Sepharose™ 6MB, and Superose™ 6 and 12, respectively.

A number of dextran-based or dextran-agarose composite materials suitable for use in the context of the present invention are also available from Amersham Pharmacia Biotech under the names Sephadex™, Superdex™ (e.g. Superdex™ 30, 75 and 200) and Sephacryl™. Products in the Sephadex™ range are prepared by cross-linking dextran with epichlorohydrin and are available in the following grades: Sephadex™ G-10, G-15, G-25, G-50, G-75, G-100, G-150 and G-200, the degree of cross-linking decreasing with increasing G number. Products in the Sephacryl™ range are prepared by cross-linking allyl-dextran with N,N'-methylene-bisacrylamide, and include Sephacryl™ S-100, S-200, S-300, S-400, S-500 and S-1000; the latter six products differ with respect to their range of pore size and particle size distribution. Products in the Superdex™ range are prepared by cross-linking allyl-dextran with agarose derivatives of various compositions.

Polyalkylene glycols and derivatives thereof, including, in particular, polyethylene glycols (PEG), i.e. condensation polymers of ethylene glycol having the general formula HOCH₂(CH₂OCH₂)ₙCH₂OH or H(OCH₂CH₂)ₙOH and typically having average molecular weights in the range from 200 to 6000. A number of PEG's (including PEG's of average molecular weight 400, 600, 1500, 4000 and 6000, respectively) are available under various names (e.g. Macrogol™, PEG™, Carbowax™, Nycoline™, Pluracol E™, Poly-G™, Polyglycol E™, Solbase™) from a variety of commercial sources. PEG's are generally soluble in or miscible with water, as well as in ethanol and a number of other organic solvents, including aromatic hydrocarbons. The analogous polypropylene glycols [having the general formula H(OC₃H₆)ₙOH], the lower molecular weight members of which are soluble in water, are also of relevance in the context of the invention. Relevant derivatives of such polyalkylene glycols include partially etherified derivatives, e.g. derivatives in which one of the terminal hydroxy groups has been converted to a lower alkyl ether group, such as a methyl ether group.

Such polymers can readily be immobilized to support materials, thereby producing substrates that can subsequently be activated and then functionalized or derivatized with macrocyclic metal ion binding chelating ligands by procedures according to the present invention.

Polyvinyl Polymers, including polyvinyl alcohols - i.e. hydroxylic polymers normally produced by hydrolysis ("alcoholysis") of various molecular weight fractions of polyvinyl acetate, typically by base or acid hydrolysis - and derivatives thereof. The degree of "alcoholysis" may be varied by either allowing the hydrolysis of acetate ester groups in polyvinyl acetate to proceed to substantial completion, or by stopping it at a desired degree of alcoholysis. Polyvinyl alcohols are normally commercially available in four molecular weight ranges, viz. ca. 250,000-300,000 (termed super-high viscosity), ca. 170,000-ca. 220,000 (termed high-viscosity), ca. 120,000-150,000 (termed medium-viscosity) and ca. 25,000-ca. 35,000 (termed low-viscosity). In general, the lower the molecular weight of polyvinyl alcohols, the higher is their water sensitivity or ease of water solubility; however, the degree of alcoholysis also plays a role with regard to the water-solubility and other properties of polyvinyl alcohols. Polyvinyl alcohols within all of the above-outlined categories are or relevance in the context of the present invention, as are, for example, ether derivatives thereof, such as methyl ether derivatives.

Other polyvinyl polymer materials of interest include materials such as the Toyopearl™ HW range of porous, semi-rigid spherical gel particles designed for medium- and low-pressure liquid chromatography. Such materials, after activation and functionalization/derivatization, provide another option for the preparation of IMAC sorbents of relevance in the context of the invention. Toyopearl™ HW gels (obtainable from Tosoh Corp, Yamaguchi, Japan, and other suppliers) are synthesized from hydrophilic vinyl polymer containing exclusively C, H and O atoms. Available grades (differing with respect to particle and pore sizes) include Toyopearl™ HW-40, HW-40C, HW-40F, HW-40S, HW-50, HW-50F, HW-50S, HW-55, HW-55F, HW-55S, HW-65F, HW-65S and HW-75F.

Polyacrylamides and derivatives thereof, including composite materials based on polyacrylamide and agarose, such as Ultrogel™ AcA gels (composite polyacrylamide-agarose gel in bead form, available from, e.g., Amersham Pharmacia Biotech). The Ultrogel™ AcA gel range includes AcA 22, AcA 34, AcA 44 and AcA 54, where the number refers to the percentage of acrylamide and agarose, i.e., AcA 22 contains 2% acrylamide and 2% agarose. Activation of hydroxylic groups of these support materials provides an avenue to the preparation of IMAC sorbents.

Surface-modified silicas, including glycidylpropoxy-modified porous silica, such as LiChroSpher™ Diol (E. Merck, Darmstadt, Germany), Toyosoda™ TSKSW3000 (Tosoh Corp., Yamaguchi, Japan); amino-propyl-modified silica, prepared by reaction (in the presence of a suitable catalyst) of aminopropyldiethoxysilane with silicas of appropriate pore size and appropriate average diameter; and mercaptopropylsilicas, prepared by reaction (in the presence of a suitable catalyst) of mercaptopropyldiethoxysilane with silicas of appropriate pore sizes and appropriate average diameters. Alternatively, dextran modified or butadiene-vinyl copolymer modified silicas of appropriate pore sizes and appropriate average diameters can be employed as the chromatographic support materials. "Naked" porous silicas suitable for such derivatization and subsequent modification to generate the respective novel IMAC sorbents can readily be obtained from a variety of suppliers, including E. Merck, (Darmstadt, Germany), Tosoh Corporation, Yamaguchi, Japan), Eka-Nobel AB (Göteborg, Sweden) and Grace Davison GmbH (Worms, Germany).

Surface-modified metal oxides, including glycidylpropoxy-modified porous zirconias, titanias or aluminas, as well as modifications/variants thereof based on the respective metal oxide "doped" with a second metal oxide; amino-propyl-modified zirconia, titania or alumina, prepared by reaction (in the presence of a suitable catalyst) of aminopropyldiethoxysilane with the zirconia, titania or alumina of appropriate pore size and appropriate average diameter; and mercaptopropyl-modified zirconia, titania or alumina, prepared by reaction (in the presence of a suitable catalyst) of mercaptopropyldiethoxysilane with the zirconia, titania or alumina of appropriate pore size and appropriate average diameter. Alternatively, dextran modified or butadiene-vinyl copolymer modified zirconia, titania or alumina of appropriate pore sizes and average diameters can be employed as the chromatographic support materials. "Naked" porous zirconia, titania or alumina suitable for such derivatization and subsequent modification to generate the respective novel IMAC sorbents can readily be obtained from a variety of suppliers, including YMC Co. Ltd. (Kyoto, Japan), Grace GmbH (Worms, Germany) and BioSepra Corp. (Paris, France).

Well suited polymer substrates in the context of the invention include agaroses, dextrans and derivatives thereof, e.g. materials selected among those outlined above.

In one aspect of the invention, the cyclic, metal ion coordinating ligand group in a functionalized polymer substrate according to the invention is derived from a heterocycle chosen among: triazacycloalkanes and -cycloalkenes; and tetraazacycloalkanes and -cycloalkenes. Among such functionalized polymer substrates, particularly well suited cyclic, metal ion coordinating ligand groups include groups derived from a heterocycle chosen among the following.
1,4,7-triazacyclononane;
1,4,7-triazacyclodecane;
1,4,8-triazacycloundecane;
1,5,9-triazacyclododecane;
1,4,7,10-tetraazacyclododecane;
1,4,7,10-tetraazacyclotridecane;
1,4,7,11-tetraazacyclotetradecane;
1,4,8,11- tetraazacyclotetradecane;
1,4,8,12-tetraazacyclopentadecane; and
1,5,9,13-tetraazacyclohexadecane.

In relation to this latter aspect of the invention, a hydrogen atom in one or more ring -CH₂- groups or - CH= groups (and/or, in some instances, in a ring -NH- group) in one of the above cyclic, metal ion coordinating ligand groups of the tri- or tetraazacycloalkane or -cycloalkene type may optionally be substituted with a substituent selected among optionally substituted lower alkyl groups and optionally substituted aryl groups; further examples of optional substituents appropriate for substituting a hydrogen atom in, in particular, a ring -CH₂- or -CH= group in a metal ion coordinating ligand groups of the tri- or tetraazacycloalkane or -cycloalkene type include optionally substituted lower alkoxy groups. The optional substituent(s) on the lower alkyl group, lower alkoxy group or aryl group in question may optionally comprise one or more metal ion coordinating donor atoms, such as one or more O or S donor atoms.

The term "lower alkyl" as employed in the context of the present invention in intended to designate any linear (straight-chain), branched or cyclic alkyl group having from 1 to 6 carbon atoms. Examples of linear alkyl groups are methyl, ethyl, propyl, butyl, pentyl and hexyl; examples of branched alkyl groups are isopropyl, iso-butyl, sec-butyl, tert-butyl, isopentyl and isohexyl; examples of cyclic alkyl groups are cyclopropyl, cyclobutyl, cyclopentyl and cyclohexyl. In general, linear or branched lower alkyl groups having from 1 to 3 carbon atoms (i.e. methyl, ethyl, propyl and isopropyl) will be well suited in the context of the invention. Suitable optional substituents on lower alkyl groups in the context of the invention include halogen, hydroxy, lower alkoxy and optionally substituted aryl.

The term "lower alkoxy" as employed in the context of the present invention in intended to designate any linear, branched or cyclic alkoxy group having from 1 to 6 carbon atoms. Examples of linear alkoxy groups are methoxy, ethoxy, propoxy, butoxy, pentoxy and hexoxy; examples of branched alkoxy groups are isopropoxy, sec-butoxy, tert-butoxy, isopentoxy and isohexoxy; examples of cyclic alkoxy groups are cyclopropyloxy, cyclobutyloxy, cyclopentyloxy and cyclohexyloxy. In general, linear or branched lower alkoxy groups having from 1 to 3 carbon atoms (i.e. methoxy, ethoxy, propoxy and isopropoxy) will be well suited in the context of the invention.

In the present context, the term "aryl" is intended to designate any aromatic group and includes both carbocyclic and heterocyclic aromatic groups. Examples thereof are phenyl, naphthyl, pyridyl, tetrazolyl, thiazolyl, imidazolyl, indolyl, quinolinyl, pyrimidinyl, thiadiazolyl, pyrazolyl, oxazolyl, isoxazolyl, thienyl, furanyl or oxadiazolyl. Suitable optional substituents on aryl groups in the context of the invention include halogen, amino, hydroxy, lower alkyl and lower alkoxy.

The term "halogen" designates Cl, F, Br or I.

With regard to the optionally substituted carboxy(lower alkyl) or optionally substituted phosphono(lower alkyl) group covalently attached to at least one of the ring N atoms of the cyclic, metal ion coordinating ligand group, carboxymethyl (-CH₂COOH) and phosphonomethyl [-CH₂PO(OH)₂], respectively, have proved to be very suitable. With cyclic, metal ion coordinating ligand groups of the triazacycloalkane or-cycloalkene type, mentioned above, it appears to be advantageous that at least two (i.e. two or three) of the three ring N atoms have an optionally substituted carboxy(lower alkyl) or optionally substituted phosphono(lower alkyl) group (e.g. a carboxymethyl or phosphonomethyl group) covalently attached thereto. In the case of metal ion coordinating groups of the tetraazacycloalkane or -cycloalkene type, mentioned above, it appears to be advantageous that at least two (i.e. two, three or four) of the four ring N atoms have an optionally substituted carboxy(lower alkyl) or optionally substituted phosphono(lower alkyl) group (e.g. a carboxymethyl or phosphonomethyl group) covalently attached thereto.

Suitable optional substituents on the lower alkyl moiety in such optionally substituted carboxy(lower alkyl) or optionally substituted phosphono(lower alkyl) groups (e.g. optional substituents on the -CH₂moiety of a carboxymethyl or phosphonomethyl group) include optionally substituted aryl, i.e. aryl (e.g. phenyl) and substituted aryl [e.g. (lower alkyl)phenyl, such as methylphenyl, ethylphenyl, propylphenyl, isopropylphenyl, cyclopropylphenyl, cyclobutylphenyl, cyclopentylphenyl or cyclohexylphenyl, where the lower alkyl group on the phenyl ring may be in any position (i.e. 2-, 3- or 4- position) relative to the carbon atom bearing the carboxy group or phosphono group.

It is generally advantageous that the functionality in a functionalized polymer substrate according to the present invention is covalently attached to the polymer substrate by means of a linker or spacer group X, the group X being attached to a ring nitrogen atom of the cyclic, metal ion coordinating ligand group. The linker or spacer group X may be any suitable type of linker or spacer, but will typically be one which may be derived from a bifunctional organic compound (e.g. an organic compound having at least two reactive functional groups selected from groups such as carboxyl, thiol, aminopropyl, halogen and epoxy) by reaction with, on the one hand, an appropriate reactive functionality on the polymer substrate and, on the other hand, the appropriate reactive functionality - in this case a substituted amino group (-NH-) in the ring - of a cyclic, metal ion coordinating ligand group. A type of linker or spacer group X which is generally very useful in the context of the present invention is one which can be derived from epichlorohydrin by reaction of the halogen end thereof with, e.g., an hydroxy group on the surface of the polymer substrate in question and then reaction of the epoxy group thereof with a substituted amino group in a cyclic ligand group.

In particularly useful embodiments of functionalized polymer substrates of the invention the linker or spacer group X is a group derivable from epichlorohydrin by reaction thereof with the polymer substrate in the form of an agarose or agarose derivative, and subsequent reaction of the resulting product with a ring -NH- group of the cyclic, metal ion coordinating ligand group which becomes bound to X.

As is apparent from the disclosure herein, an important feature of the materials (functionalized polymer substrates) employed according to the invention to isolate and purify a desired protein (protein of interest) is the presence, in the material, of a metal ion which itself is bound coordinatively to a cyclic ligand group in the functionality, and which in turn is capable of binding coordinatively, and suitably selectively, to donor atoms in the amino acid residues of the oligopeptide "tag" part of a fusion protein in which the oligopeptide "tag" is attached to the amino acid sequence of the protein of interest. A further aspect of the invention thus relates to a functionalized polymer substrate as described above, in which at least one of the cyclic ligand groups in the functionality has a metal ion coordinated thereto. Functionalized polymer substrates disclosed and described herein are particularly well suited to coordination of certain divalent (2+-charged) or trivalent (3+-charged) metal ions, notably metal ions chosen among Ca²⁺, Mag²⁺, Zn²⁺ and Fe³⁺. As illustrated by working examples provided herein (*vide infra*), Ca²⁺ is a versatile metal ion in this connection.

As already indicated briefly, a further aspect of the invention relates to a process for preparing a functionalized polymer substrate according to the invention, the process comprising the steps of: selecting a polymer substrate having a reactive functional group capable of undergoing a first reaction with a first functional group of a bifunctional reagent having a first and a second functional group; the first reaction in question resulting in covalent bond formation between the polymer substrate and the bifunctional reagent; the second functional group of the resulting covalently bound reagent being subsequently capable of undergoing a second reaction with a reactive ring -NH- group present in a species comprising at least one cyclic, metal ion coordinating ligand group which comprises at least 3 nitrogen donor atoms in the ring of the cyclic group, at least one of the nitrogen atoms in question having an optionally substituted carboxy(lower alkyl) or phosphono(lower alkyl) group covalently attached thereto; and the second reaction resulting in covalent bond formation between the species in question and the covalently bound reagent;
reacting the polymer substrate with the bifunctional reagent; and
reacting the resulting covalently bound reagent with the species in question.

In relation to the latter process according to the invention, the polymer substrate employed, and the cyclic, metal ion coordinating ligand group in the reactive species employed in the process, may be chosen among those already discussed above in connection with functionalized polymer substrates according to the invention. The bifunctional reagent employed will typically be a bifunctional organic compound, e.g. an organic compound having at least two reactive functional groups chosen among groups such as carboxyl, thiol, aminopropyl, halogen and epoxy. Epichlorohydrin is particularly useful as a bifunctional reagent for a number of types of polymer substrate, including polysaccharides and derivatives thereof having surface hydroxyl groups.

As will be apparent from the discussion above in relation to functionalized polymer substrates according to the invention, the reactive species containing the cyclic, metal ion coordinating ligand group will suitably be one which gives rise to a functionality (in the resulting functionalized polymer substrate product) of one of the types described above. Thus, appropriate reactive species for use in the process of the invention will then include species containing one or more cyclic, metal ion coordinating ligand groups having a reactive ring -NH- group and being derived from heterocycles chosen among: triazacycloalkanes and -cycloalkenes or among tetraazacycloalkanes and -cycloalkenes, e.g. species containing one or more cyclic, metal ion coordinating ligand groups having a reactive ring -NH- group and being derived from heterocycles chosen among:
1,4,7-triazacyclononane;
1,4,7-triazacyclodecane;
1,4,8-triazacycloundecane;
1,5,9-triazacyclododecane;
1,4,7,10-tetraazacyclododecane;
1,4,7,10-tetraazacyclotridecane;
1,4,7,11-tetraazacyclotetradecane;
1,4,8,11- tetraazacyclotetradecane;
1,4,8,12-tetraazacyclopentadecane; and
1,5,9,13-tetraazacyclohexadecane.

The considerations above (in the context of functionalized polymer substrates of the invention) with regard to the optionally substituted carboxy(lower alkyl) or phosphono(lower alkyl) group covalently attached to at least one of the ring N atoms of a cyclic, metal ion coordinating ligand group likewise apply to the above-described process of the invention. Thus, carboxymethyl (-CH₂COOH) and phosphonomethyl [-CH₂PO(OH)₂] are very suitable as optionally substituted carboxy(lower alkyl) groups and phosphono(lower alkyl) groups, respectively.

As will also be apparent from the foregoing discussion, agaroses and agarose derivatives (such as Sepharose™ products as described above) are well suited as polymer substrates in the context of the above-described process according to the invention. A well-suited bifunctional reagent will then be epichlorohydrin, and it may be advantageous in this connection to further incorporate a reducing agent, such as sodium borohydride, in the reaction mixture when reacting the polymer substrate with epichlorhydrin.

The scope of the present invention further encompasses functionalized polymer substrates obtained or obtainable by a process as described above for preparing a functionalized polymer substrate.

In addition, the scope of the present invention also encompasses a process for preparing a functionalized polymer substrate which is in accordance with the invention, and which further comprises a metal ion coordinated to at least one of the cyclic, metal ion coordinating groups therein, the process comprising contacting a functionalized polymer substrate according to the invention with an aqueous solution of an inorganic salt [e.g. a nitrate, halide (fluoride, chloride, bromide or iodide), sulfate, perchlorate, tetrafluoroborate, hexafluorophosphate or phosphate salt] or organic salt [e.g. a carboxylate (such as formate, acetate, propanoate or benzoate), tetraphenylborate or sulphonate salt] of the metal ion in question (e.g. one of the metal ions already mentioned above). A metal ion-containing functionalized polymer substrate obtained or obtainable by such a process is also within the scope of the present invention.

**Selection and preparation of metal ion coordinating (chelating) ligands having strong affinity for hard metal ions (and metal ions of borderline hardness)**: An important and valuable application of functionalized polymer substrates as defined in the context of the present invention is the use of a metal ion containing embodiment thereof in the purification of a protein, the protein in question being in the form of a fusion protein wherein the protein of interest is fused at its amino or carboxy terminus to an oligopeptide "tag", such as an Asp-containing oligopeptide according to the invention. In addition, the simultaneous fusion of two molecules of a protein or polypeptide of interest, or alternatively two different proteins or polypeptides of interest attached at their amino- or carboxy- terminus, respectively, to an oligopeptide "tag", such as an Asp-containing oligopeptide according to the invention, generates a new fusion protein structure whereby the oligopeptide "tag" is located at an endo-position (i.e. an internal position) linking the two molecules of the protein(s) or polypeptide(s) of interest.

Not all chelating ligands fulfil the requirements to be a suitable ligand for Immobilised Metal Affinity Chromatography (IMAC). The chelating ligands serve two aims: (a) they fix the metal ion to a solid support and (b) they modulate the metal affinity binding and thus the strength and affinity specificity of the adsorption centre.¹ Ideally, the chelating ligand should form stable complexes with the metal ions so that no metal ions are released into the solvent phase or transferred to the biomolecules during adsorption and desorption of these molecules. At the same time it should also leave at least one, and preferably two or more coordination sites of the metal ion available for protein binding.

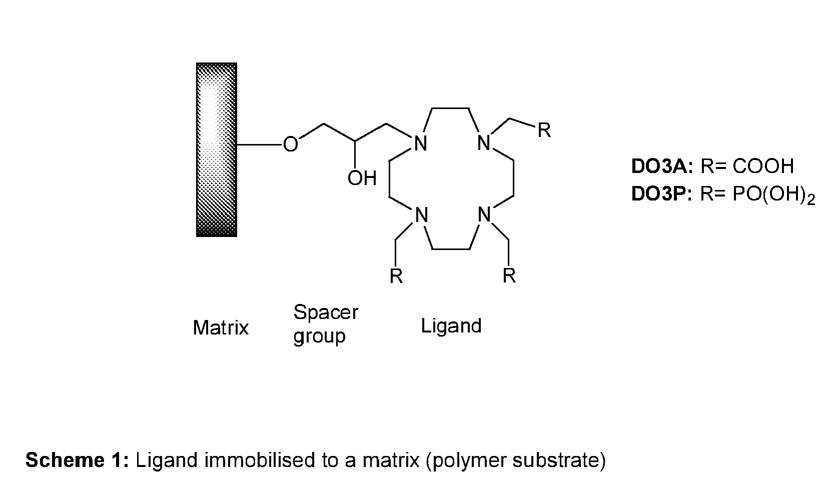

Six ligands, based on the macrocycles cyclen and tacn, and containing carboxymethyl or phosphonomethyl pendant arms, have been synthesised on the basis of published methods²⁻⁴. These ligands are 1,4,7-tris(carboxymethyl)-1,4,7,10-tetraazacyclododecane (DO3A), 1,7,-bis(phosphonomethyl)-1,4,7,10-tetraazacyclododecane (DO2P), 1,4,7-tris(phosphonomethyl)-1,4,7,10-tetraazacyclododecane (DO3P), 1-(carboxymethyl)-1,4,7-triazacyclononane (T1A), 1,4-bis(carboxymethyl)-1,4,7-triazacyclononane (T2A) and 1,4-bis(phosphonomethyl)-1,4,7-triazacyclononane (T2P). As shown in Scheme 1, a secondary amine group enables attachment to an activated gel (polymer substrate), generating the IMAC support (functionalized polymer substrate). The stability constants of the corresponding Ca²⁺- complexes, reported by Burai *et al* ⁵ for DO2P (log *K*_{Ca(II)L}=12.8) and by Chang^{5a} for DO3A (log *K*_{Ca(II)L}=10.72), are relatively high, and such ligands are thereby desirable for use in IMAC systems of the type in question as they minimize metal ion "leakage" during protein purification, and minimize metal ion transfer from the ligand to the protein. Furthermore, since a maximum coordination number of 10 for calcium has been reported, coordination sites will be available for protein binding.

The three macrocycles DO2P, DO3A and DO3P (see Scheme 2) were prepared by literature methods²⁻⁴ and characterised by NMR and mass spectroscopy (MS). Preparation of DO3A and DO3P was achieved in high yields, and scaling-up of the reaction to obtain larger quantities (e.g. gram quantities or more) was successful. The NMR and MS data obtained, as well as yields obtained, were very similar to those previously reported²⁻⁴.

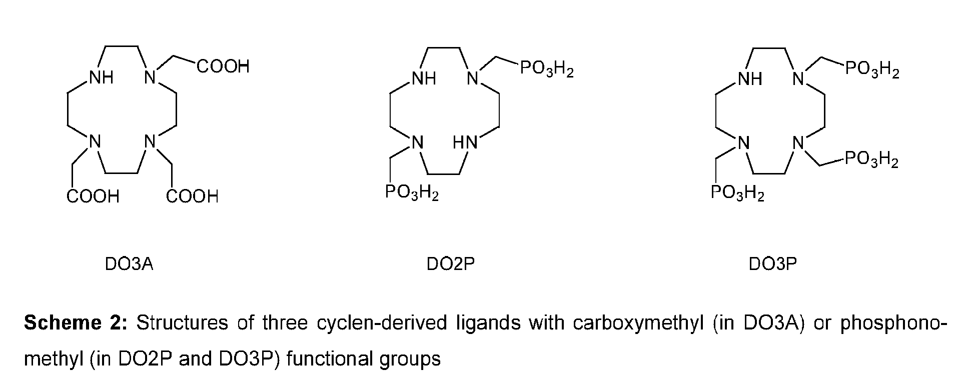

The synthesis of DO2P required some optimisation in order to increase the yield and efficiency. Scaling up to multi-gram quantities could not be achieved. The yields and NMR data obtained were similar to those described in the literature^{5,6}. Suitable crystals of the DO2P ligand were obtained for X-ray analysis, but severe disorder of the macrocyclic ring prevented satisfactory refinement of the crystal structure. Nevertheless, it confirmed the presence of both phosphonomethyl arms lying on the same side of the plane defined by the macrocyclic ring. Crystals of the free ligand DO3P have been obtained and the structure confirmed by x-ray crystallography. The ORTEP plot and a space-filling model of the ligand DO3P are shown below in Scheme 3a and 3b, respectively:

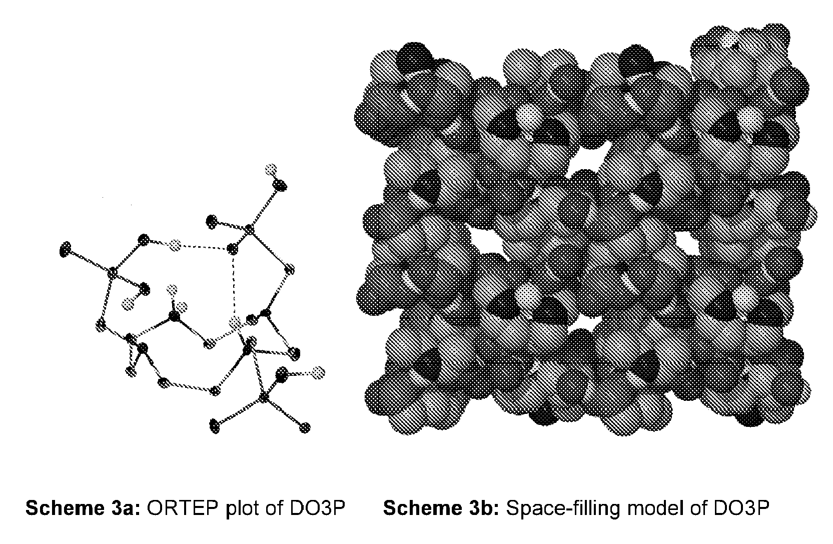

Among triaza- and tetraazacycloalkanes of types mentioned above, cyclen is commercially available, whereas tacn and cyclam were synthesised. In the case of tacn, the multi-step synthesis was carried out by the well-established method by Richman-Atkins⁷ based on the cyclisation of selectively protected polyamines and diols. The synthesis can be achieved in high yields and high purity. The next step involves the synthesis of derivatized macrocycles containing carboxymethyl or phosphonomethyl side-arms (examples of which are shown in Scheme 4, below, for tacn):

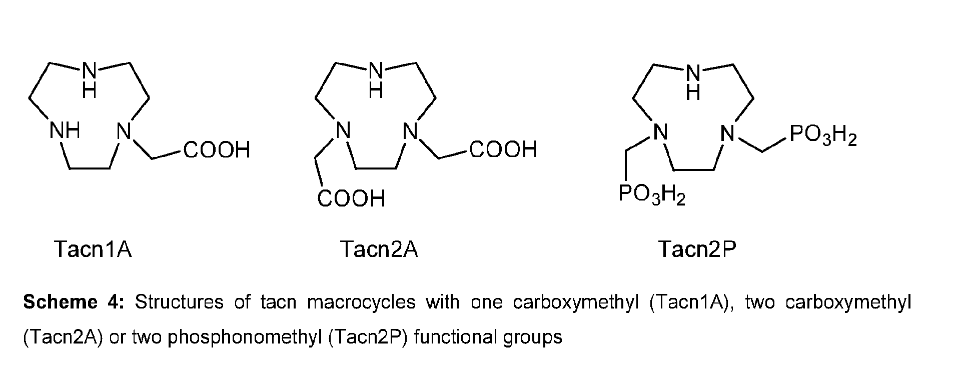

These latter tacn-derived ligands are expected to be suitable for complexation of Fe³⁺ in IMAC systems, since the maximum coordination number of Fe³⁺ is six. An underivatized secondary amine (-NH-) is used for immobilisation to the chromatographic support matrix (polymer substrate), and since the ligands occupy a maximum of five sites in the Fe³⁺ coordination sphere (in the case of immobilised Tacn2A and Tacn2P) there will be still at least one coordination site available for protein binding.

The corresponding tetraaza ligand cyclam was synthesized by a method based upon that described by Parker⁸ for the preparation of cyclen. The first precursor for the cyclam synthesis (N,N',N",N"'-tetratosyl-1,5,8,12-tetraazadodecane) was synthesised using two different methods^{7,8}. Both methods led to products with identical NMR spectra and melting points. The structure was also confirmed by mass spectroscopy. Cyclam-derived macrocycles containing two or three carboxymethyl and two or three phosphonomethyl arms, respectively (Scheme 5), can be synthesized in a manner analogous to that described for the tacn and cyclen molecules, and function as 12-membered ring analogues of tacn and cyclen systems with carboxymethyl and phosphonomethyl pendant arms as detailed above.

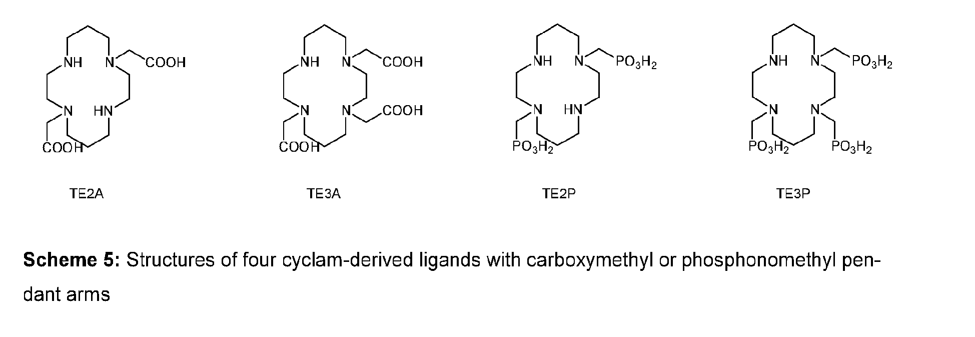

Owing to the increased ring size compared to tacn rings, the derivatized cyclam ligands may also be used for the complexation of larger metal ions, such as calcium or magnesium.

**Immobilization (covalent attachment) of ligands to Polymer substrate (matrix) and of metal ions to immobilized ligand**: IMAC in the context of the present invention uses immobilized metal complexes, produced by binding (complexation) of metal ions to chelating ligands attached to a chromatographic matrix or support, to capture proteins containing specific metal-binding sites. The adsorption of these proteins is based on the coordination interaction between the immobilized metal ion and electron- donor groups from the protein surface. In the preparation of IMAC sorbents according to the invention, the generation of an immobilized metal complex typically involves several steps. In a typical procedure, the chelating ligand is firstly attached to a chromatographic matrix (polymer substrate) which has previously been activated using a difunctional reagent, e.g. epichlorohydrin or 1,4-butanediol diglycidyl ether (bisoxirane). The second step involves the complexation of a metal ion to the immobilized chelating ligand, normally by treatment with an aqueous solution of a salt (e.g. chloride or nitrate) of the metal ion in question. The resulting metal chelate complex is able to interact with a protein molecule, dissolved in a liquid mobile phase, such that electron-donor groups of amino acid residues situated at the surface of the protein (e.g. an imidazole group of a histidine residue) displace weakly coordinated solvent ligands (e.g.H₂O) and form a coordinative bond with the immobilized metal ion. Finally, the elution of the bound protein is achieved by reducing the pH of the mobile phase, or alternatively by using competitive ligands which displace the protein from the coordination site on the metal ion.

The selectivity of IMAC separations can be influenced by the choice of metal ion, chelating ligand and/or solvent conditions, or by modification of the target protein.

The following describes the immobilization process with reference to the ligands DO3A and DO3P (*vide supra*) and to a polymer substrate in the form of a commercially available agarose derivative, Sepharose™ 6FF, which is well suited as a substrate in the context of the invention. Each of the ligands in question is designed to contain a free amine group for attachment to a solid support matrix. The resulting immobilised (im) ligands (im-DO3P, im-DO3A) have been tested for their ability to bind to calcium ions.

### Immobilisation of ligands to Sepharose™ 6FF

The macrocycles may be immobilized on Sepharose™ using the standard epoxy-activation method previously described. The first step involves the treatment of the Sepharose™ gel (polymer substrate) with epichlorohydrin under basic conditions to produce an epoxy-activated gel. The attachment of the ligands is then achieved through reaction of the nucleophilic secondary amine group in the ring of the ligand with the electrophilic epoxide surface group of the epoxy-activated gel. As shown in Scheme 6 (below), the reaction with the epoxy group introduces a spacer group that allows the immobilised ligand more conformational freedom to interact with the protein. As the ligand is the only N-containing component of the system, the amount of immobilised ligand on the matrix can be calculated by nitrogen-analysis. As shown in Table 1 below, the highest surface coverage (ligand density) is obtained with immobilization of cyclen *per se*. In the experiments performed to this end, the DO3A ligand became immobilized to a reasonable level (optimum ca. 300 µmol/g dry gel), whereas the DO3P ligand did not become immobilized as well. This may have been due to longer storage of the epoxy-activated gel prior to the ligand immobilization for the DO3P. Alternatively, the low ligand coverage may be due to the greater steric bulk of the ligand preventing access to all epoxy groups available for interaction. Moreover, the phosphono groups of the DO3P ligand are more negatively charged than the carboxy groups of the DO3A ligand at the pH employed in the immobilization (pH 12), and electrostatic repulsion effects may thus inhibit immobilization coverage.

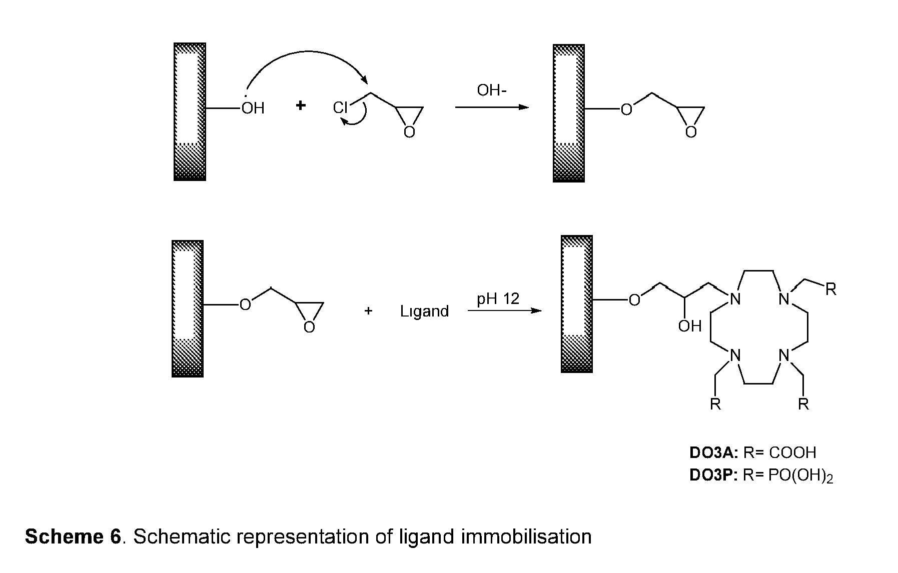

**Table 1: Nitrogen content and calculated ligand density for immobilised ligands**

| Immobilised ligand | %N (w/w) | Ligand density (µmol/g dry gel) |
|---|---|---|
| Tacn | 1.28 | 305 |
| Cyclen | 1.81 | 323 |
| Tacn1A | 1.19 | 285 |
| Tacn2A | 1.24 | 289 |
| DO3A | 1.19 | 212 |
| DO3P | 0.52 | 93 |

### Immobilization of metal ions

The immobilisation of the metal ions was achieved by incubating the gels with solutions of the corresponding metal-ion chlorides for a period of one hour at room temperature. To ensure full deprotonation of the acid groups of the pendant arms of the *im*-ligands, the pH of the metal-ion solutions was raised to pH 10 (except in the case of Fe³⁺, in order to avoid formation of hydroxo or oxo species). The amount of metal ion bound was determined by atomic adsorption spectroscopy (AAS) measurements, summarised in Table 2 below. Illustrative of the methods employed, the immobilisation of Ca²⁺ ions was thus achieved by incubating the gels (*im*-DO3A, *im*-DO3P) with CaCl₂ solution (pH 10) for a period of one hour at room temperature.

**Table 2: Mⁿ⁺ binding to various ligand derivatives**

| | Tacn | Cyclen | Tacn1A | Tacn2A | DO3A | DO3P |
|---|---|---|---|---|---|---|
| Ligand density (µmol/g dry gel) | 305 | 323 | 285 | 289 | 212 | 93 |
| Ca²⁺ content (µmol/g dry gel) | * | * | 95 | 139 | 197 | 84 |
| Zn²⁺ content (µmol/g dry gel) | * | * | 240 | 220 | 129 | 140 |
| Fe³⁺ content (µmol/g dry gel) | * | * | 228 | 201 | 103 | 191 |

These results indicate that in most cases the stoichiometry of metal-to-ligand binding is 1.1. In the case of DO3P, the Zn²⁺ and Fe³⁺ uptake by the gel was found to be approx. 2.1, whereas in the case of T1A (i.e. Tacn1A) and T2A (i.e. Tacn2A) the Ca²⁺-to-ligand stoichiometry was found to be approx. 1.2.

**Design of peptide tag sequences for binding to immobilized hard metal ions (or metal ions of borderline hardness):** It should be noted here that the meaning of the terms "hard", "hardness" and "soft" as employed in the present specification in relation to metal ions is that according to R.G. Pearson (see, e.g., S.F.A. Kettle, Coordination Chemistry, Thomas Nelson and Sons Ltd., 1969, pp. 48-49). At one end of the scale, "hard" metal ions are those which parallel the proton with respect to their attachment to ligands, are small, are often of high charge, and which lack valence shell electrons which are easily distorted or removed, hard metal ions include, e.g., Ca²⁺, Mg²⁺, Mn²⁺, Cr³⁺, La³⁺ and Fe³⁺. At the other end of the scale, "soft" metal ions are large, of low charge or have valence shell electrons which are easily distorted or removed, soft metal ions include, e.g., Cu⁺, Ag⁺ and Cd²⁺. Metal ions whose properties place them on the borderline between hard and soft - i.e. are of "borderline hardness" - include, e.g., Zn²⁺, Fe²⁺, Co²⁺, Ni²⁺ and Cu²⁺.

Hard metal ions have the strongest affinity for hard Lewis bases. As such, amino acid residues in proteins containing carboxylate groups (Asp, Glu) can bind strongly to hard metal ions, such as Ca²⁺. In order to obtain specific interactions of recombinant proteins with a hard metal ion, a specific fusion tag is required. These hard metal ion binding tags are ideally short peptide tags that can be introduced into the protein using recombinant genetic techniques. To design, for example, specific Ca²⁺-binding tags, naturally occurring Ca²⁺-binding proteins were analysed for their amino acid sequence involved in Ca²⁺ binding. These sequences bind Ca²⁺ with high affinity and as such have the potential to provide a basis for tag design. Examples of such proteins are the calcium-binding proteins calmodulin (CaM), troponin C (TnC), and parvalbumin (Parv). All these proteins bind Ca²⁺ with high affinities¹³.

| | | |
|---|---|---|
| CaM. | four binding sites | log *Kₛ* = 6 |
| TnC. | four binding sites | log *Kₛ* = 7 |
| Parv. | two binding sites | log *Kₛ* = 9 |

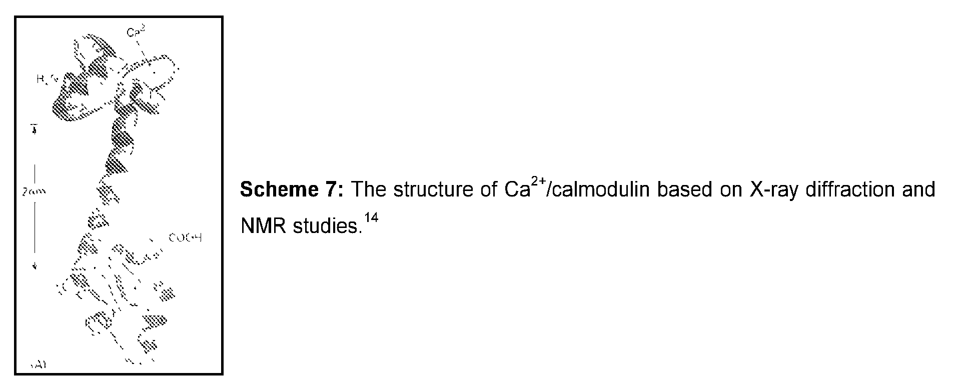

Scheme 7 shows the structure of calmodulin with the four calcium-binding sites. It is generally accepted that two of the four binding sites have lower affinity for Ca²⁺, and that the two other sites have slightly higher affinity for Ca²⁺.

The structural studies show that the molecule has a "dumbbell" shape, with two globular ends connected by a long, exposed α-helix. Each end has two Ca²⁺ -binding sites, each with a loop of 12 amino acid residues in which residues such as aspartic acid and glutamic acid residues (shown in bold print in Table 3, below) form electrostatic/coordinate bonds with calcium. The two Ca²⁺-binding sites in the carboxyl-terminal part of the molecule (Domains III, IV) have a ten-fold higher affinity for calcium than those in the amino-terminal part (Domains I, II). The four calcium-binding domains with their sequences are shown in Table 3.

**Table 3: Sequence and position of the calcium-binding sites of calmodulin**

| Ca²⁺-Binding Domains | Amino acid numbers | Amino Acid Sequence |
|---|---|---|
| I | 20 to 31 | -**Asp**-Lys-**Asp**-Gly-**Asp**-Gly-**Thr**-Ile-**Thr**-Thr-Lys-**Glu**- |
| II | 56 to 67 | -**Asp**-Ala-**Asp**-Gly-**Asn**-Gly-**Thr**-Ile-**Asp**-Phe-Pro-**Glu**- |
| III | 93 to 10 | -**Asp**-Lys-**Asp**-Gly-**Asp**-Gly-**Val**-Ile-**Ser**-Ala-Ala-**Glu**- |
| IV | 129 to 140 | -**Asp**-Ile-**Asp**-Gly-**Asp**-Gly-**Gln**-Val-**Asn**-Tyr-Glu-**Glu**- |

Based on these sequences (Table 3), eight peptide sequences (Scheme 8) were designed that potentially bind Ca²⁺ with varying affinities. In peptides 1-3, there are 5 amino acid residues sites involved in Ca²⁺ binding (indicated in **bold** in Scheme 8). Given that the affinity of the tags for Ca²⁺ needs to be lower than the affinity of Ca²⁺ for the ligand (to prevent loss of Ca²⁺ from the *im*-ligand), shorter tags (Tag 4-Tag 6) were also designed to reduce the binding affinity of Ca²⁺ for the tag.

| | | |
|---|---|---|
| Tag 1: | M**D**A**D**G**N**G**T**IDFA**E**F | (SEQ. ID No. 1) |
| Tag 2: | M**D**I**D**G**D**G**H**INYE**E**F | (SEQ. ID No. 2) |
| Tag 3: | M**D**V**D**G**S**G**T**IGSS**E**L | (SEQ. ID No. 3) |
| Tag 4: | M**D**V**D**R**S**G**T**IGSS**E**L | (SEQ. ID No. 4) |
| Tag 5: | M**D**A**D**G**N** | (SEQ. ID No. 5) |
| Tag 6: | M**D**I**D**G**D** | (SEQ. ID No. 6) |
| Tag 7: | M**D**V**D**G**S** | (SEQ. ID No. 7) |
| Tag 8: | M**D**V**D**R**S** | (SEQ. ID No. 8) |

**Scheme 8:** Amino acid sequence of calcium binding tags.

In addition to the above "tag" sequences, the invention also encompasses variants of such sequences wherein one or more amino acid residues (e.g. a single amino acid residue) are replaced with (i.e. substituted by) a different amino acid residue, particularly with an amino acid residue or amino acid residues which is of similar chemical functionality to the the replaced amino acid residue. In this context, the term "similar chemical functionality" is intended to indicate that the replaced amino acid residue and the amino acid residue replacing it are closely related with respect to polarity, with respect to polarizability, with respect to number of acidic and basic substituents and/or with respect to structural analogy or homology. Thus, with respect to non-highlighted amino acid residues (i.e. residues not shown in bold font) in tag sequences among those shown in Scheme 8 (above) it will appropriate, for example, to replace:
an Ala (A) residue (a non-polar residue) with a Gly (G) residue (likewise non-polar), or *vice versa*;
a Phe (F) residue (containing a phenyl ring) with a Tyr (Y) residue (differing from Phe by virtue of a 4-hydroxy group on the phenyl ring), or *vice versa*;
a "nonpolarizable" Val (V), Leu (L) or Ile (I) residue with one of the other two residues or with another "nonpolarizable" residue, such as Pro (P) or Met (M).

With respect to highlighted residues (shown in bold font) in tag sequences among those shown in Scheme 8 (above) it will be appropriate, for example, to replace:
an Asp (D) residue (an "acidic" residue) with a Glu (E) residue (likewise "acidic"), or *vice versa*;
an Asn (N) residue with a Gln (Q) residue.

Oligopeptides comprising one or more of the amino acid sequences shown in Scheme 8, and/or one or more substitution variants of such sequences, also constitute an aspect of the present invention. In such oligopeptides, the "tag" sequences in question (or substitution variants thereof) may be identical or different.

It will be understood that a peptide (oligopeptide) "tag" may comprise, in addition to one of the amino acid sequences shown above, or a substitution variant thereof as described above, one or more additional amino acid residues, such as from 2 to 6 or more additional amino acid residues, attached at the C- or N-terminal end of the oligopeptide "tag" (e.g. at the C- or N-terminal end of one of the eight sequences (Tag 1 - Tag 8) shown in Scheme 8.

Employing well known molecular biological approaches and techniques, preferably based on polymerase chain reaction (PCR) procedures, the introduction of an oligopeptide "tag" according to the invention into a protein of interest is achievable at the amino terminal, at the carboxyl terminal or at internal (endo-) positions. As a consequence, implementation of a C-terminally located oligopeptide "tag" still enables the described IMAC procedures to be employed with the present macrocyclic chelating ligand systems. Moreover, the oligopeptide "tag" (e.g. one of those listed above in Scheme 8) according to the invention can be simultaneously fused to two molecules of the protein or polypeptide of interest, or alternatively to two different proteins or polypeptides of interest, at their amino- or carboxyterminus, respectively, thereby forming a new fusion protein structure whereby the oligopeptide "tag" is located at an endo-position (i.e. at an internal position) linking the two molecules of the protein(s) or polypeptide(s) of interest.

**Synthesis of peptides containing tag sequences**: In order to test the suitability of the latter tag sequences for use in IMAC systems in the context of the invention, peptides having the sequences of Tag 1 - Tag 8, respectively, were synthesized by Fmoc-based solid-phase peptide synthesis. This procedure is based on the sequential addition of L-amino acids which are side-chain protected and N-terminal Fmoc-protected (Fmoc = 9-fluorenylmethyloxycarbonyl) to an insoluble polymeric support. After removal of the N-terminal protecting group, the next Fmoc-protected amino acid is added with the addition of coupling reagents (HOBt, HBTU, DIPEA). The deprotection and coupling step is repeated until the final crude peptide is obtained. Standard TFA cleavage methods may be employed to cleave the product from the resin and remove side-chain protecting groups from the crude peptide. The crude peptides were purified by preparative Reverse-Phase High-Pressure Liquid Chromatography (RP-HPLC), e.g. using gradient elution with increasing concentrations of acetonitrile.

Still further aspects of the invention include the following:
a polypeptide which is a fusion protein comprising a protein of interest fused at its amino terminus or carboxy terminus to at least one oligopeptide among those referred to above in connection with SEQ. IDs Nos. 1-8, or among those referred to above in connection with substitution variants of such oligopeptides; as already discussed (*vide supra*), fusion proteins of the type in question include fusion proteins wherein two molecules of a protein or polypeptide of interest, or alternatively two different proteins or polypeptides of interest, are attached simultaneously at their amino- or carboxy- terminus, respectively, to one and the same oligopeptide (i.e. oligopeptide "tag"), suitably an oligopeptide according to the invention, so as to form a fusion protein structure in which the oligopeptide (i.e. the "tag") is located at an endo-position (i.e. an internal position) linking the two molecules of the protein(s) or polypeptide(s) of interest; in fusion protein structures of this type, the amino acid sequence of the oligopeptide "tag" may suitably be flanked by one or more enzymatic or chemical cleavage sites;
a polynucleotide construct, such as a vector, encoding such a polypeptide;
a polypeptide obtainable by cultivating a host cell (e.g. a prokaryote such as *Escherichia coli*) comprising such a polynucleotide construct in an appropriate growth medium under conditions allowing expression of the polypeptide, and recovering the polypeptide from the culture medium;
a host cell, e.g. a prokaryote cell (e.g. a strain of *Escherichia coli*), comprising such a polynucleotide construct; and
a method for producing a polypeptide of the type in question, the method comprising cultivating a host cell of the type in question in an appropriate growth medium under conditions allowing expression of the polypeptide, and recovering the polypeptide from the culture medium;

Yet another aspect of the invention relates to a method for purifying a protein of interest, the method comprising the steps of:
contacting a protein sample which contains: a polypeptide which is a fusion protein comprising the protein of interest fused at its amino terminus or carboxy terminus to at least one oligopeptide (i.e. oligopeptide "tag") according to the invention (i.e. a fusion protein of one of the types already mentioned above, including a fusion protein in which the oligopeptide (i.e. the "tag") is situated in an *endo* position as already discussed); and other (extraneous) proteins; with a metal ion-containing functionalized polymer substrate according to the invention under conditions whereby the polypeptide (fusion protein) binds to the metal ion-containing functionalized polymer substrate so as to form a complex therewith;
washing the complex with a buffer solution to remove the other (extraneous) proteins; and
eluting the bound polypeptide from the washed complex.

The latter method may further comprise a step wherein the oligopeptide (the "tag") is cleaved from the polypeptide or protein of interest, e.g. by chemical means or by means of an enzyme, e.g. an endo- or exo-peptidase.

The invention also encompasses a purified protein obtained or obtainable by the latter method.

**Expression of target proteins with affinity tags**: Recombinant DNA techniques can be used to introduce a fusion tag to a protein for use in subsequent purification. Affinity tags are short fragments of DNA that code for an amino acid sequence which has a strong binding affinity for a metal ion. These tags (c-DNA) are ligated (joined) to the c-DNA of the target recombinant protein at the N- or C-terminus. The resulting tagged fusion protein can be expressed (produced) and subsequently purified from crude cell extract using IMAC systems since the attached tag binds selectively or specifically to the immobilized metal ions. Elution of the fusion protein can be achieved by applying a continuously decreased pH gradient. Alternatively, a chelating agent such as EDTA can be added to the mobile phase to elute the protein. If required, the affinity tag can be removed from the target protein (post purification) using chemical or enzymatic methods.

Polypeptides or proteins (polypeptides or proteins of interest) which are of relevance in relation to the purification methodology taught in the context of the present invention include the following: mammalian proteins, such as, e.g., growth hormone, including human growth hormone and bovine growth hormone; growth hormone releasing factor; parathyroid hormone; thyroid stimulating hormone; lipoproteins; α-1-antitrypsin; insulin A-chain; insulin B-chain; proinsulin; follicle stimulating hormone; calcitonin; luteinizing hormone; glucagon; clotting factors, such as Factor VII (including Factor VIIa), Factor VIII, Factor IX, tissue factor, and von Willebrands factor; anti-clotting factors such as Protein C; atrial natriuretic factor; lung surfactant; a plasminogen activator, such as urokinase or tissue-type plasminogen activator (t-PA); bombazine; thrombin; tumor necrosis factor-α and -β; enkephalinase; RANTES (regulated on activation normally T-cell expressed and secreted); human macrophage inflammatory protein (MIP-1-α); serum albumin, such as human serum albumin; mullerian-inhibiting substance; relaxin A-chain; relaxin B-chain; prorelaxin; mouse gonadotropin-associated peptide; DNase; an activin (e.g. activin A, activin B, activin C, activin D or activin E); an inhibin (e.g. inhibin A or inhibin B); vascular endothelial growth factor (VEGF); receptors for hormones or growth factors; an integrin; protein A or D; rheumatoid factors; a neurotrophic factor such as bone-derived neurotrophic factor (BDNF), neurotrophin-3, -4, -5, or -6 (NT-3, NT-4, NT-5 or NT-6), or a nerve growth factor such as NGF-β; platelet-derived growth factor (PDGF); fibroblast growth factor, such as aFGF and bFGF; epidermal growth factor (EGF); transforming growth factor (TGF), such as TGF-α and TGF-β, including TGF-β1, TGF-β2, TGF-α3, TGF-β4 and or TGF-β5; insulin-like growth factor-I and -II (IGF-I and IGF-II); des(1-3)-IGF-I (brain IGF-I); insulin-like growth factor binding proteins; CD proteins, such as CD3, CD4, CD8, CD19 or CD20; erythropoietin (EPO); thrombopoietin (TPO); osteoinductive factors; immunotoxins; a bone morphogenetic protein 1-7 (BMP 1-7); an interferon, such as interferon-α, -β or -γ; colony stimulating factors (CSFs), e.g., M-CSF, GM-CSF or G-CSF; interleukins (ILs), e.g., IL-1 to IL-12; superoxide dismutase; T-cell receptors; surface membrane proteins; decay accelerating factor (DAF); a viral antigen, such as, for example, a portion of the AIDS envelope; transport proteins; homing receptors; addressins; regulatory proteins; immunoadhesins; antibodies; and biologically active fragments or variants of any of the above-listed polypeptides or proteins.

The term "polynucleotide" as employed herein denotes a single- or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules. The length of a polynucleotide molecule is given in terms of nucleotides (abbreviated "nt") or base pairs (abbreviated "bp"). The term "nucleotide" is used for both single- and double-stranded molecules, where the context permits. When the term is applied to double-stranded molecules it is used to denote overall length and will be understood to be equivalent to the term "base pairs". It will be recognized by those skilled in the art that the two strands of a double-stranded polynucleotide may differ slightly in length and that the ends thereof may be staggered as a result of enzymatic cleavage; thus all nucleotides within a double-stranded polynucleotide molecule may not be paired. Such unpaired ends will in general not exceed 20 nt in length.

The term "host cell" as employed herein denotes any cell, including a hybrid cell, in which heterologous DNA can be expressed. Typical host cells include, but are not limited to, bacterial cells, insect cells, yeast cells and mammalian cells, including human cells, such as BHK, CHO, HEK, and COS cells.

The term "vector" as employed herein denotes any nucleic acid entity capable of amplification in a host cell. Thus, the vector may be an autonomously replicating vector, i.e. a vector that exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated. The choice of vector will often depend on the host cell into which it is to be introduced. Vectors include, but are not limited to plasmid vectors, phage vectors, viruses or cosmid vectors. Vectors usually contain a replication origin and at least one selectable gene, i.e. a gene that encodes a product which is readily detectable, or the presence of which is essential for cell growth.

In the present specification, amino acid residues are represented using abbreviations approved by the IUPAC-IUB Commission on Biochemical Nomenclature (CBN). With respect to amino acids, those represented by the following abbreviations are in the naturally occurring L-form. Further, the left and right ends of an amino acid sequence of a peptide are, respectively, the N- and C-termini unless otherwise specified.

| Abbreviations for amino acid residues | | |
|---|---|---|
| Amino acid | Three-letter code | One-letter code |
| Glycine | Gly | G |
| Proline | Pro | p |
| Alanine | Ala | A |
| Valine | Val | V |
| Leucine | Leu | L |
| Isoleucine | Ile | I |
| Methionine | Met | M |
| Cysteine | Cys | C |
| Phenylalanine | Phe | F |
| Tyrosine | Tyr | Y |
| Tryptophan | Trp | W |
| Histidine | His | H |
| Lysine | Lys | K |
| Arginine | Arg | R |
| Glutamine | Gln | Q |
| Asparagine | Asn | N |
| Glutamic Acid | Glu | E |
| Aspartic Acid | Asp | D |
| Serine | Ser | S |
| Threonine | Thr | T |

The enzyme classification employed in the present specification with claims is in accordance with *Recommendations (1992) of the Nomenclature Committee of the International Union of Biochemistry and Molecular Biology*, an updated version of which (including supplements) is available on the world wide web at http.//www.chem.qmw.ac.uk/iubmb/enzyme/.

### EMBODIMENTS OF THE INVENTION

1. A polymer substrate functionalized with a functionality comprising at least one cyclic, metal ion coordinating ligand group which comprises at least 3 nitrogen donor atoms in the ring of said cyclic group, at least one of said nitrogen atoms having an optionally substituted carboxy(lower alkyl) or optionally substituted phosphono(lower alkyl) group covalently attached thereto.
2. A functionalized polymer substrate according to embodiment 1, wherein said polymer is substantially water-insoluble.
3. A functionalized polymer substrate according to embodiment 1 or 2, wherein said polymer is selected from the group consisting of: polysaccharides and derivatives thereof; polyalkylene glycols and derivatives thereof; polyvinyl alcohols and derivatives thereof; polyacrylamides; surface-modified silicas; and surface-modified metal oxides.
4. A functionalized polymer substrate according to any one of the preceding embodiments, wherein said polymer is selected from the group consisting of: agarose and derivatives thereof; dextran and derivatives thereof; and cellulose and derivatives thereof.
5. A functionalized polymer substrate according to any one of the preceding embodiments, wherein said cyclic, metal ion coordinating ligand group is derived from a heterocycle chosen among:
triazacycloalkanes and -cycloalkenes; and
tetraazacycloalkanes and -cycloalkenes.
6. A functionalized polymer substrate according to embodiment 5, wherein said cyclic, metal ion coordinating ligand group is derived from a heterocycle chosen among:
1,4,7-triazacyclononane;
1,4,7-triazacyclodecane;
1,4,8-triazacycloundecane;
1,5,9-triazacyclododecane;
1,4,7,10-tetraazacyclododecane;
1,4,7,10-tetraazacyclotridecane;
1,4,7,11-tetraazacyclotetradecane;
1,4,8,11- tetraazacyclotetradecane;
1,4,8,12-tetraazacyclopentadecane; and
1,5,9,13-tetraazacyclohexadecane.
7. A functionalized polymer substrate according to any one of embodiments 1-7, wherein said optionally substituted carboxy(lower alkyl) group is carboxymethyl.
8. A functionalized polymer substrate according to any one of embodiments 1-7, wherein said optionally substituted phosphono(lower alkyl) group is phosphonomethyl.
9. A functionalized polymer substrate according to any one of embodiments 1-8, wherein said functionality is covalently attached to said polymer substrate by means of a linker or spacer group X, said group X being attached to a ring nitrogen atom of said cyclic, metal ion coordinating ligand group.
10. A functionalized polymer substrate according to embodiment 9, wherein said polymer substrate is an agarose, and said linker or spacer group X is a group derivable from epichlorohydrin by reaction thereof with agarose and subsequent reaction of the resulting product with a ring -NH- group of said cyclic, metal ion coordinating ligand group.
11. A functionalized polymer substrate according to any one of the preceding embodiments, further comprising a metal ion coordinated to at least one of said cyclic ligand groups in said functionality.
12. A functionalized polymer substrate according to embodiment 11, wherein said coordinated metal ion is a divalent or trivalent metal ion.
13. A functionalized polymer substrate according to embodiment 12, wherein said metal ion is selected from the group consisting of Ca²⁺, Mg²⁺, Zn²⁺ and Fe³⁺.
14. A process for preparing a functionalized polymer substrate according to embodiment 1, comprising the steps of:
selecting a polymer substrate having a reactive functional group capable of undergoing a first reaction with a first functional group of a bifunctional reagent having a first and a second functional group,
   said first reaction resulting in covalent bond formation between said polymer substrate and said bifunctional reagent,
   said second functional group of the resulting covalently bound reagent being subsequently capable of undergoing a second reaction with a reactive ring -NH- group present in a species comprising at least one cyclic, metal ion coordinating ligand group which comprises at least 3 nitrogen donor atoms in the ring of said cyclic group, at least one of said nitrogen atoms having an optionally substituted carboxy(lower alkyl) or optionally substituted phosphono(lower alkyl) group covalently attached thereto,
   said second reaction resulting in covalent bond formation between said species and said covalently bound reagent;
reacting said polymer substrate with said bifunctional reagent; and
reacting said resulting covalently bound reagent with said species.
15. A process according to embodiment 14, wherein said polymer is substantially water-insoluble.
16. A process according to embodiment 14 or 15, wherein said polymer is selected from the group consisting of: polysaccharides and derivatives thereof; polyalkylene glycols and derivatives thereof; polyvinyl alcohols and derivatives thereof; polyacrylamides; surface-modified silicas; and surface-modified metal oxides.
17. A process according to any one of embodiments 14-16, wherein said polymer is selected from the group consisting of: agarose and derivatives thereof; dextran and derivatives thereof; and cellulose and derivatives thereof.
18. A process according to any one of embodiments 14-17, wherein said cyclic, metal ion coordinating ligand group is derived from a heterocycle chosen among:
triazacycloalkanes and -cycloalkenes; and
tetraazacycloalkanes and -cycloalkenes.
19. A process according to any one of embodiments 14-18, wherein said cyclic, metal ion coordinating ligand group is derived from a heterocycle chosen among:
1,4,7-triazacyclononane;
1,4,7-triazacyclodecane;
1,4,8-triazacycloundecane;
1,5,9-triazacyclododecane;
1,4,7,10-tetraazacyclododecane;
1,4,7,10-tetraazacyclotridecane;
1,4,7,11-tetraazacyclotetradecane;
1,4,8,11- tetraazacyclotetradecane;
1,4,8,12-tetraazacyclopentadecane; and
1,5,9,13-tetraazacyclohexadecane.
20. A process according to any one of embodiments 14-19, wherein said optionally substituted carboxy(lower alkyl) group is carboxymethyl.
21. A process according to any one of embodiments 14-19, wherein said optionally substituted phosphono(lower alkyl) group is phosphonomethyl.
22. A process according to any one of embodiments 14-21, wherein said polymer substrate is an agarose, and said bifunctional reagent is epichlorohydrin.
23. A process according to embodiment 22, wherein a reducing agent is incorporated in the reaction mixture when reacting said polymer substrate with said bifunctional reagent.
24. A process according to embodiment 23, wherein said reducing agent is sodium borohydride.
25. A functionalized polymer substrate obtainable by a process according to any one of embodiments 14-24.
26. A process for preparing a functionalized polymer substrate according to any one of embodiments 1-10 or 25 and further comprising a metal ion coordinated to at least one of said cyclic groups, the process comprising contacting a functionalized polymer substrate according to any one of embodiments 1-10 or 25 with an aqueous solution of an inorganic or organic salt of said metal ion.
27. A metal ion-containing functionalized polymer substrate obtainable by a process according to embodiment 26.
28. An oligopeptide comprising an amino acid sequence selected from the group consisting of.

| | |
|---|---|
| M**D**A**D**G**N**G**T**IDFA**E**F | (SEQ. ID No. 1) |
| M**D**I**D**G**D**G**H**INYE**E**F | (SEQ. ID No. 2) |
| M**D**V**D**G**S**G**T**IGSS**E**L | (SEQ. ID No. 3) |
| M**D**V**D**R**S**G**T**IGSS**E**L | (SEQ. ID No. 4) |
| M**D**A**D**G**N** | (SEQ. ID No. 5) |
| M**D**I**D**G**D** | (SEQ. ID No. 6) |
| M**D**V**D**G**S** | (SEQ. ID No. 7) |
| M**D**V**D**R**S** | (SEQ. ID No. 8) |

and further consisting of. variants of said sequences wherein one or more amino acid residues in a sequence chosen among said sequences are replaced with an amino acid residue of similar chemical functionality to the the replaced amino acid residue.
29. An oligopeptide according to embodiment 28, wherein.
an Ala (A) residue in a sequence chosen among said sequences is replaced with a Gly (G) residue, or *vice versa*,
a Phe (F) residue in a sequence chosen among said sequences is replaced with a Tyr (Y) residue, or *vice versa*,
a Val (V), Leu (L) or Ile (I) residue in a sequence chosen among said sequences is replaced with a different amino acid residue chosen among Val (V), Leu (L), Ile (I), Pro (P) and Met (M),
an Asp (D) residue in a sequence chosen among said sequences is replaced with a Glu (E) residue, or *vice versa*, and/or
an Asn (N) residue in a sequence chosen among said sequences is replaced with a Gln (Q) residue.
30. A polypeptide which is a fusion protein comprising a protein molecule of interest fused at its amino terminus or carboxy terminus to at least one oligopeptide according to embodiment 28 or 29.
31. A polypeptide according to embodiment 30, wherein the amino terminus and the carboxy terminus of the amino acid sequence of said oligopeptide are each fused to a protein molecule of interest.
32. A polypeptide according to embodiment 31, wherein the amino acid sequence of said oligopeptide is flanked by cleavage sites selected from the group consisting of enzymatic and chemical cleavage sites.
33. A polypeptide according to embodiment 31 or 32, wherein said two protein molecules of interest are molecules of the same protein.
34. A polypeptide according to embodiment 31 or 32, wherein said two protein molecules of interest are different.
35. A polypeptide obtainable by cultivating a host cell comprising a polynucleotide construct encoding a polypeptide according to any one of embodiments 29-34 in an appropriate growth medium under conditions allowing expression of said polypeptide, and recovering said polypeptide from the culture medium.
36. A polypeptide according to embodiment 35, wherein said host cell is a strain of *Escherichia coli*.
37. A polynucleotide construct encoding a polypeptide according to any one of embodiments 29-34.
38. A polynucleotide construct according to embodiment 37, which is a vector.
39. A host cell comprising a polynucleotide construct according to embodiment 37 or 38.
40. A host cell according to embodiment 39, which is a strain of *Escherichia coli*.
41. A method for producing a polypeptide according to any one of embodiments 29-36, the method comprising cultivating a host cell as defined in embodiment 39 or 40 in an appropriate growth medium under conditions allowing expression of said polypeptide, and recovering said polypeptide from the culture medium.
42. A method for purifying a protein of interest, the method comprising the steps of:
contacting a protein sample containing a polypeptide according to any one of embodiments 29-36, comprising said protein of interest together with other proteins, with a metal ion-containing functionalized polymer substrate according to any one of embodiments 11-13 or 27 under conditions whereby said polypeptide binds to said metal ion-containing functionalized polymer substrate so as to form a complex therewith;
washing the complex with a buffer solution to remove said other proteins; and eluting the bound polypeptide from the washed complex.
43. A method according to embodiment 42, further comprising a step wherein said oligopeptide is cleaved from said polypeptide.
44. A method according to embodiment 43, wherein said oligopeptide is cleaved from said polypeptide by enzymatic means.
45. A method according to embodiment 44, wherein an endopeptidase or exopeptidase is employed to cleave said oligopeptide from said polypeptide.
46. A purified protein obtainable by a method according to any one of embodiments 42-45.

### EXPERIMENTAL SECTION

| **ABBREVIATIONS** | |
|---|---|
| | |
| APS | ammonium persulfate |
| BES | N, N-Bis-(2-Hydroxyethyl)-2-aminoethanesulfonic acid |
| CAPS | 3-(cyclohexylamino)-1-propanesulfonic acid |
| CHES | 2-(cyclohexylamino)-ethanesulfonic acid |
| Cyclam | 1,4,8,12-tetraazacyclopentadecane |
| Cyclen | 1,4,7,10-tetraazacyclododecane |
| Da | Dalton |
| DIPEA | 1,3-diisopropylethylamine |
| DMF | N,N-dimethylformamide |
| DO3A | 1,4,7-tris(carboxymethyl)-1,4,7,10-tetraazacyclododecane |
| DO2P | 1,7,-bis(phosphonomethyl)-1,4,7,10-tetraazacyclododecane |
| DO3P | 1,4,7-tris(phosphonomethyl)-1,4,7,10-tetraazacyclododecane |
| EDT | 1,2-ethanedithiol |
| GST | glutathione S-transferase |
| HBTU | O-benzotriazole-N,N,N',N'-tetramethyl-uronium-hexafluorophosphate |
| HEPES | 4-(2-hydroxyethyl) piperazine-1-ethanesulfonic acid |
| HOBt | N-(hydroxybenzotriazole) |
| IMAC | immobilised metal ion affinity chromatography |
| *im* | immobilised |
| *im*-Ca²+ | immobilised calcium ions |
| *im*-Fe³+ | immobilised ferric ions |
| *im*-Mⁿ⁺ | immobilised metal ions |
| *im*-Ca²+ -DO3A | immobilised metal ion complex derived from DO3A and Ca²⁺ ions |
| *im*-Ca2⁺-DO3P | immobilised metal ion complex derived from DO3P and Ca²⁺ ions |
| I PTG | isopropyl-β-D-thiogalactoside |
| MES | 2-(N-morpholino)-ethanesulfonic acid |
| MOPS | 3-(N-morpholino)-propanesulfonic acid |
| PAGE | polyacrylamide gel electrophoresis |
| PBS | phosphate-buffered saline |
| SDS | sodium dodecyl sulfate |
| Tacn1A or T1A | 1-(carboxymethyl)-1,4,7-triazacyclononane |
| Tacn2A or T2A | 1,4-bis(carboxymethyl)-1,4,7-triazacyclononane |
| Tacn2P or T2P | 1,4-bis(phosphonomethyl)-1,4,7-triazacyclononane |
| Tacn | 1,4,7-triazacyclononane |
| Tris | tris(hydroxymethyl)-aminomethane |
| TFA | trifluoroacetic acid |

### Metal ion coordinating ligands

### Synthesis of 1,7-bis(phosphonomethyl)-1,4,7,10-tetraazacyclododecane (DO2P)

The multi step synthesis of DO2P was performed using previously reported method^{5,6}.

### 1,7-bis(Benzyloxycarbonylmethyl)-1,4,7,10-tetraazacyclododecane (I)

Cyclen (0.5g) was dissolved in H₂O (20ml) and the pH adjusted to 3.0 by the addition of 6M HCl, followed by the addition of dioxan (15ml). Benzylchloroformate (12.8ml) was dissolved in dioxan (10ml) and the solution was added to the mixture through a syringe whilst the pH was maintained between 2-3 by the addition of 2M NaOH (about 20ml). After 16 hours the addition was complete and the solvent was evaporated under reduced pressure. The white solid residue obtained was extracted with diethyl ether to give a clear oil.

**Yield:** 0.7 g. 1.57mmol, 54%

**¹H-NMR** (300 MHz, CDCl₃). δ(ppm) 2.80 (br m, NCH₂, 8H), 3.42 (br m, NCH₂, 8H), 5.15 (s, OCH₂, 4H), 7.34 (m, C₆H₅, 10H).

### 1,7-bis(Benzyloxycarbonylmethyl)-4,10-bis(diethoxyphosphonomethyl)-1,4,7,10-tetraazacyclododecane (II)

As described in the literature by Burai et. al⁵, to **I** (0.7g) was added triethyl phosphite (0.63g, 20% excess) and the mixture cooled to 0°C on ice. Paraformaldehyde (0.1 g, 10% excess) was added slowly over a period of 30 minutes and the resulting mixture warmed to room temperature. Stirring for 2 days at room temperature, followed by stirring for one day at 40°C gave a clear yellow oil, which was then kept under high vacuum at 40-50°C for several hours to remove volatile impurities. The resulting clear oil was used for the next synthetic step without further purification.

**Yield:** 0.5g, 0.67mmol, 43%

**³¹P-NMR** (CDCl₃/H₃PO₄): δ(ppm) 26.62

**¹H-NMR** (CDCl₃): δ(ppm). 7.30 (m, aromatic protons, 10 H), 5.13 (s, benzyl protons, 4H), 4.05 (m POCH₂CH₃, 8H), 3.4 (br, CONCH₂CH₂NCH₂P, 8H), 2.85 (br, CONCH₂CH₂NCH₂P, and NCH₂P, 12H), 1.30 (t, POCH₂CH₃, 12H).

### 1,7,-bis(Phosphonomethyl)-1,4,7,10-tetraazacyclododecane (DO2P) (III)

The crude oil (**II**) was dissolved in 5M HCl (50ml) and refluxed for 24 hours. After cooling to room temperature the mixture was extracted with diethyl ether and the diethyl ether evaporated under reduced pressure. The solid residue was dissolved in absolute ethanol (25 ml) and diethyl ether (25ml) was added. A white precipitate formed immediately, and was filtered off and dried under vacuum.

**Yield:** 0.1 g, 0.28mmol, 42%

**Melting point:** 285-290°C

**³¹P-NMR:** δ(ppm) 22.26

**Anal.** Calcd. for C₁₀H₂₆N₄O₆P₂ · 1.65H₂O · 1 HCl: C, 28.17; H, 7.25; N 12.57; found: C, 28.17; H, 7.16; N, 13.14

**¹H-NMR** (D₂O, pH∼ 2.0):δ(ppm) 2.91 3.0, 3.3 (br, ring protons, 16H), 2.90 (d, N*CH*₂P, 4H)

### Crystallographic analysis.

DO2P was recrystallised from an EtOH/H₂O solution followed by the addition of diethyl ether. Suitable crystals for X-ray crystallography had formed upon standing at 4°C over two weeks. Crystal parameters are presented in Table I.

**Table I: Crystal Data for DO2P**

| | |
|---|---|
| Chemical formula | C₁₀H₂₆N₄O₆P₂ |
| | |
| Crystal system | Triclinic |
| | |
| Symmetry used in scale pack | C2 |
| | |
| a, Å | 30.347(6) |
| | |
| b, Å | 16.196(4) |
| | |
| c, Å | 8.373 |
| | |
| αdeg | 90.000 |
| | |
| βdeg | 97.939(4) |
| | |
| γdeg | 90.000 |
| | |
| V, A³ | 4076.06 |
| | |
| Mosaicity (°) | 0.816 |

### Synthesis of 1,4,7-tris(carboxymethyl)-1,4,7,10-tetraazacyclododecane (DO3A)

Synthesis is performed in four steps according to published method^{3,9}(Scheme A). 1,4,7,10-Tetraazatricyclotridecane (**1**) was prepared according to the method of Atkins *et al.* ⁹, and Steps 2-4 were carried out following the method of Dischino *et al* ³.

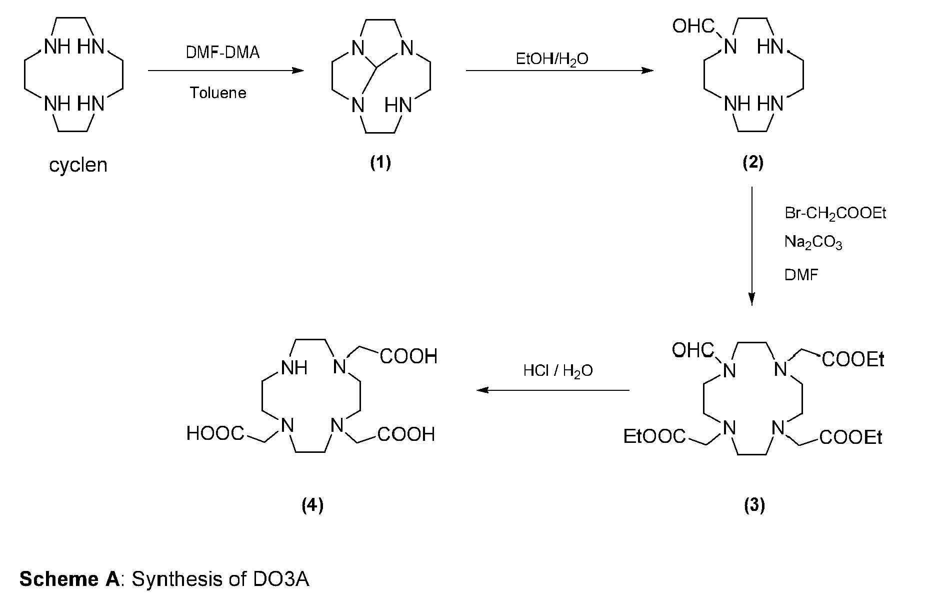

### 1,4,7,10-Tetraazatricyclotridecane (1)

Cyclen (5g, 29mmol) was dissolved in toluene (50ml), and dimethylformamide dimethylacetal (3.45g, 29mmol) was added. The mixture was stirred at 80°C overnight, after which the toluene was removed under reduced pressure. The crude oil was purified by distillation under high vacuum.

**Yield:** 4g, 22.03mmol, 76%

**¹H-NMR** (CDCl₃): δ(ppm) 1.66 (s, N-H, 1 H); 2.88 (s, CH₂- CH₂, 16H); 4.44 (s, C-H, 1 H).

### 1-Formyl-1,4,7,10-tetraazacyclododecane (2)

1,4,7,10-Tetraazatricyclotridecane (1; 4g, 22.03mmol) was cooled down to 4°C, and an EtOH/H₂O solution (50ml, chilled to -20°C) was added. The resulting reaction mixture was allowed to slowly warm to room temperature and then stirred under nitrogen for 12 hours. The reaction mixture was concentrated in vacuo, dissolved in acetonitrile (50ml), and concentrated again. This process was repeated three times to remove traces of H₂O. The pale yellow oil was dried under vacuum at room temperature overnight to yield a white hydroscopic solid.

**Yield:** 3.95g, 19.8mmol, 90%

**¹H-NMR** (D₂O): δ(ppm) 2.5-2.8 (m, CH₂, 12H); 3.55 (m, CH₂, 4H); 8.15(s, CHO, 1 H).

### 1,4,7-tris(Ethoxycarbonylmethyl)-10-formyl-1,4,7,10 tetraazacyclododecane (3)

A solution of 3.95g (19.8mmol) of **2** in DMF (20ml, cooled to 4°C) was stirred under nitrogen, and *tert-*butyl bromoacetate (15.43g, 79.1mmol) was added over a period of 30 minutes. The temperature of the reaction mixture was maintained at 30°C for further 35 minutes. Anhydrous sodium carbonate (8.4g, 80mmol) dissolved in water (80ml) was then added to the above reaction mixture, which was stirred for 30 minutes. After addition of toluene (20ml) the mixture was allowed to stir for an additional 4 hours. This mixture was then transferred to a separating funnel und the layers were separated. The toluene layer was extracted three times with 1 M Na₂CO₃ (50ml), followed by 0.8M HCl (1x25ml) and finally with H₂O (25ml). The aqueous layers were combined and the pH adjusted to 9.4 using Na₂CO₃. These combined layers were then extracted twice with dichloromethane (DCM; 50ml), and the DCM layers were combined and dried over Na₂CO₃. The organic layer was concentrated in vacuo to yield **3** as a viscous pale yellow oil.

**Yield:** 9.32g, 17.2mmol, 87%

**¹H-NMR** (CDCl₃): δ(ppm) 1.45 (s, CH₃, 27H); 2.68-3.0 (m, CH₂, 12H); 3.2-3.6 (m, CH₂, 10H); 8.05(s, CHO, 1 H).

### 1,4,7-tris(Carboxymethyl)-1,4,7,10-tetraazacyclododecane DO3A (4)

To 2g (3.7mmol) of **3** was added 5M HCl (100 ml), and the solution was heated to reflux under nitrogen for 6 hours. The reaction mixture was concentrated on a rotary evaporator, water (50ml) was added, and the mixture was again concentrated under vacuum. This process was repeated three times to remove traces of HCl. The resulting clear oil was then dissolved in H₂O (50ml) and the solvent removed under high vacuum (freeze drying) to give a pale yellow product.

**Yield:** 1.27g, 3.58mmol, 97%

**¹³C-NMR (D₂O):** δ(ppm) 41.3, 46.7, 48.3, 51.1, 52.1, 53.58, 167.6, 173.4

**¹H-NMR (D₂O):** δ(ppm) 2.9-3.3 (br, ring protons, 16H), 3.6 (s, CH₂COOH, 6H)

**Mass spectrum** [(ESI) m/z]: calcd for DO3A: 346; found: 347 (M+H⁺), 345 (M-H⁻).

### Synthesis of 1,4,7-tris(Phosphonomethyl)-1,4,7,10-tetraazacyclododecane (DO3P)

DO3P was prepared by literature methods^{4,9,10} as follows (Scheme B):

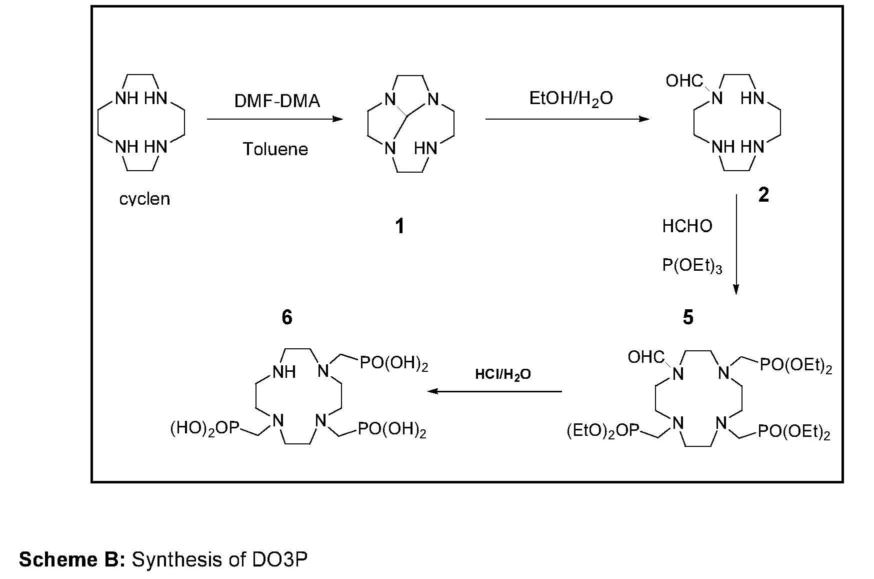

1,4,7,10-tetraazatricyclotridecane (1) and 1-formyl -1,4,7,10-tetraazacyclododecane (2) were prepared as described above for the synthesis of DO3A.

### 1,4,7-tris(Phosphonomethyl)-1,4,7,10-tetraazacyclododecane (DO3P) (6)

1-formyl-1,4,7,10-tetraazacyclododecane (2g, 10mmol) and triethylphosphite (6g, 36.1mmol, 20%excess) were placed in a round-bottom flask, and the flask was immersed in an ice bath. Paraformaldehyde (1 g, 33mmol, 10% excess) was added in small portions over a period of 30 minutes. The mixture was then allowed to warm to room temperature, and stirred for 2 days at room temperature followed by one day at 40-50°C. The clear mixture was kept under high vacuum at 50°C for several hours to remove volatile impurities. The resulting crude phosphonate ester (**5**) was hydrolysed without further purification. It was dissolved in 5M HCl (200 ml) and the mixture was refluxed for two days. The hydrochloric acid was removed by rotary evaporation to give a clear oil. It was dissolved in water (100 ml) and the solvent was removed under reduced pressure. This procedure was repeated twice more. Finally the oil was dissolved in water (925 ml), and ethanol (10 ml) was added. The product precipitated as a white solid and was filtered off, washed with water (3x20 ml) and air-dried. The crude product was recrystallised from hot water to give **6** as a fine white crystalline solid which was dried in vacuum to a constant mass.

**Yield:** 4g, 8.6mmol, 86%

**¹H-NMR(D₂O, NH₃, pH 10):** δ(ppm) 2.8 (d, *CH*₂PO(OH)², 4H,), 3.15 (br, ring protons, 14 H), 3.4 (br s, CH₂NHCH₂, 4H)

**Mass spectrum** [(ESI) m/z]: calcd for DO3P: 454; found: 455 (M+H⁺).

### Synthesis of 1,4,8,12-Tetraazacyclopentadecane (Cyclam)

### N,N',N",N"'-Tetratosyl-1,5,8,12-tetraazacyclododecane

### Method A:

1,5,8,12-Tetraazacyclododecane (10g, 57.4mmol) was dissolved in distilled water (70 ml), and sodium hydroxide (9.2g, 229.5mmol) was added slowly under stirring, ensuring the temperature did not exceed 40°C. The mixture was then cooled to 15°C and diluted with diethyl ether (70ml). Tosyl chloride (43.75g, 229.5mmol) was then added in small portions to ensure that the temperature remained at 15°C. The solution was stirred for another hour on ice and then allowed to stand overnight at room temperature. The resulting white precipitate was filtered off, washed with water and dried at 80°C for two hours. The crude product was then stirred in hot methanol for one hour, filtered off and dried under vacuum.

**Yield:** 35.4g, 44.8mmol, 78%

**¹H-NMR(d⁶ acetone):** δ(ppm) 7.73 (8H, benzyl protons), 7.4 (8H, benzyl protons), 3.18 (8H, CH₂), 2.95 (4H, CH₂) 2.42 (12H), 1.78 (4H)

**Melting point:** 125-128°C.^{19,20}

### Method B:

1,5,8,12 Tetraazacyclododecane (5g, 28.7mmol) and K₂CO₃ (8g) were dissolved in water (100ml) and heated to 80°C. Tosyl chloride (24.6g, 129.15mmol) was added in small portions over a period of 3 hours. The reaction mixture was heated and stirred overnight. The resulting clear solution was cooled to room temperature, and a precipitate was formed. The resulting white solid was filtered off and washed three times each with water, methanol and diethyl ether, and then dried under vacuum to yield a white powder.

**Yield:** 17g, 21.5mmol, 75%

**¹H-NMR(d⁶ acetone):** δ(ppm) 7.73 (8H, benzyl protons), 7.4 (8H, benzyl protons), 3.18 (8H, CH₂), 2.95 (4H, CH₂) 2.42 (12H), 1.78 (4H)

**Melting point:** 125-128°C.^{19,20}

**Mass spectrum** [(ESI) m/z]: calcd for C₃₆H₄₆N₄O₈S₄: 790; found: 791 (M+H⁺).

### Immobilization of ligands on Sepharose™ 6FF

### Preparation of epoxy-activated Sepharose 6FF

Sepharose™ 6FF (500g) was washed extensively with water, suction dried and placed in a round-bottom flask. 2M NaOH (500ml) containing NaBH₄ (1.88mg/ml) was added, and the suspension was stirred for 2 hours at room temperature. Epichlorohydrin (300ml) was then added and the suspension stirred for 5 days. The resulting epoxy-activated gel was collected by vacuum filtration, washed copiously with water (5x500ml) and stored in 20% ethanol at 4°C until used for ligand immobilisation.

### Immobilisation of DO3A

A 0.2M solution of DO3A (20 ml) was prepared and adjusted to pH 12 with 2M NaOH. This DO3A solution was added to suction-dried epoxy-activated Sepharose™ 6FF (20g), and the reaction mixture was shaken for 4 days at room temperature. The resulting immobilised DO3A gel (im-DO3A) was filtered off, washed extensively with water (5x50 ml) and stored in 20% ethanol at 4°C.

### Immobilisation of D03P

Due to the insolubility of DO3P in water, a suspension of DO3P in water (10 ml) was prepared and the pH adjusted to 12 with 2M NaOH. By thus increasing the pH, the DO3P was found to dissolve. The resulting solution was diluted to a final volume of 20ml, and suction-dried epoxy-activated Sepha-rose™ (20g) was added. The suspension was incubated for 4 days at room temperature. After this time the immobilised DO3P gel (*im*-DO3P) was collected by vacuum filtration and washed with 5x50ml of water. The gel was stored at 4°C in 20% ethanol.

### Nitrogen analysis of functionalised Sepharose sorbents

The immobilized ligand gels (*im*-DO3A, im-DO3P) were analysed for nitrogen content to determine the extent of ligand immobilisation. For each gel, approximately 10g (wet weight) of gel was collected by filtration and freeze dried in vacuo to a constant weight. The dried sorbents were weighed accurately, and the nitrogen content was analysed by Dairy Technical Services Ltd., Melbourne, Australia.

### Immobilisation of calcium and other metal ions on immobilised chelating ligands

To suction-dried immobilized ligand gels (10g wet weight) were added 0.1M CaCl₂ (or 0.1 M other metal chloride) solution, whereafter the pH was adjusted to 10 with 2M NaOH and the total volume was increased to 50 ml. The resulting mixture was incubated for one hour at room temperature with shaking. The gels were then collected by filtration and washed with water (5x50 ml). Approximately 1g (wet weight) of gel was freeze-dried in vacuo, weighed accurately and digested in 5M HCl (20 ml) for 2 hours at 50°C. The solution was diluted to exactly 50 ml with H₂O, and the Ca²⁺ (or other metal ion) content determined by atomic absorption spectroscopy (AAS) using a Varian AA-1475 atomic absorption spectrometer at a metal-specific wavelength and working range (e.g. for Ca²⁺ ions at a wavelength of 422.7nm and with a working range from 1 to 4 ppm).

### Synthesis of peptides corresponding to tag sequences

### Solid Phase Peptide Synthesis

The eight peptides with the sequences shown in Scheme 7 (*vide supra*) were synthesised using a PS3 Protein Technologies Automated Peptide Synthesiser (Rainin Instrument Co., Woburn, MA, USA). The resin (0.2mmol) (pre-coupled with the first amino acid) was loaded in a specially designed reaction vessel, mixed with DMF (10 ml) and allowed to swell for one hour at room temperature. Two-fold excesses of Fmoc-protected L-amino acids (0.4 mmol), HOBt and HBTU were weighed into separate carousel vials of the peptide synthesiser. For each peptide, the synthesis cycles per amino acid (AA) were as follows.
I Deprotection (20% piperidine in 8 ml DMF). 2x 5 min
II Washing of resin (10 ml DMF). 6 x 30 sec
III Dissolving AA/activant (7%v/v DIPEA in 8 ml DMF). 1 x 30 sec
IV AA coupling (HOBt, HBTU, DIPEA). 1 x 60 min
V Washing of resin (10 ml DMF). 6 x 30 sec
VI Return to I and couple with next amino acid

After the synthesis was completed the resin/peptide product was transferred to a plastic sinter with DMF, and washed with methanol (2x10 ml) and diethyl ether (10 ml). The crude resin/peptide product was dried in a desiccator overnight.

### Cleavage of peptide from resin

The dry resin/peptide product was transferred to a 50 ml round-bottom flask and cooled in an ice bath. The pre-prepared, chilled cleavage reagent mixture (phenol/TFA/EDT/thioanisole/water - see below) was added to the resin/peptide product, and the mixture stirred for two hours at room temperature. The mixture was then filtered, the filter washed with TFA, and the combined filtrate + washings concentrated under vacuum. Cold diethyl ether was then added and the mixture shaken vigorously. The precipitated peptide was filtered off, redissolved in 50% acetonitrile/water and freeze-dried overnight to yield a fluffy white crude product.

| | |
|---|---|
| Cleavage mixture. | 0.375g phenol |
| | 0.25 ml deionized water |
| | 0.125 ml EDT |
| | 0.25 ml thioanisole |
| | 5.00 ml TFA |

The results are summarized in Table II, below.

**Table II: Yields of crude peptides from resin**

| Peptide | Wang-Resin with first amino acid (X) attached X= | Crude resin-peptide (mg) | Amount cleaved (mg) | crude peptide (mg) | Yield (%) |
|---|---|---|---|---|---|
| Peptide 1 | Phenylalanine | 477 | 315 | 161.2 | 35.5 |
| Peptide 2 | Phenylalanine | 680 | 300 | 143.6 | 33.1 |
| Peptide 3G | Leucine | 456 | 249 | 129.8 | 30.0 |
| Peptide 3R | Leucine | 556 | 297.7 | 161.2 | 33.1 |
| Peptide 4 | Asparagine (Trt) | 366 | 252.4 | 82.3 | 27.5 |
| Peptide 5 | Aspartic Acid (tBu) | 377 | 216 | 79.6 | 23.7 |
| Peptide 6G | Serine | 401 | 265 | 82.4 | 21.7 |
| Peptide 6R | Serine | 443 | 243.3 | 102.6 | 27.0 |

### Purification of crude peptide

Preparative and analytical Reverse-Phase HPLC (RP-HPLC) were performed on a Waters Associates (Milford, MA, USA) liquid chromatography system consisting of two Model 600 solvent delivery pumps, a Rheodyne injector, a WISP Model 712 auto-sampler and an automated gradient controller. Detection was carried out using a Model 486 variable wavelength UV-detector connected to the Waters Millennium software computer. The peptides were purified by preparative RP-HPLC with different elution gradients (Table III, below) of Buffer A (0.1 % TFA) to Buffer B [90% (v/v) acetonitrile/water, 0.1 % TFA] over 1 hour with a flow-rate of 6ml/min, and with detection at a wavelength at 230 nm using a TSK-ODS-120T C-18 (300 x 21.55 mm) column from TOSOH (Tokyo, Japan). The fractions from the preparative runs of RP-HPLC were collected with a Pharmacia (Frac-100) fraction collector from Pharmacia Biotech AB (Uppsala, Sweden), and freeze-dried overnight. The purity of the collected fractions was determined by analytical RP-HPLC using a TSK-ODS-120T C-18 (150 x 4.6 mm) column from TOSOH (Tokyo, Japan) with an elution gradient of Buffer B (0 - 85%) over 25 minutes with a flow-rate of 1ml/min and with detection at 214 nm.

**Table III: Purification Results (preparative RP-HPLC) for each peptide**

| Peptide | Gradient In 60 min. | Crude Peptide (mg) | Purified peptide (mg)/ Yield (%) |
|---|---|---|---|
| Peptide 1 | 0→ 90% buffer B | 40.4 | 19.2 / 47.5 |
| Peptide 2 | 0→ 100% buffer B | 30 | 15.1 / 50.3 |
| Peptide 3G | 0→ 90% buffer B | 29.5 | 5.8 / 19.6 |
| Peptide 3R | 0→ 80% buffer B | 30.0 | 6.7 / 22.3 |
| Peptide 4 | 0→ 80% buffer B | 30.3 | 14.9 / 49.2 |
| Peptide 5 | 0→ 80% buffer B | 18.6 | 5.5 / 29.6 |
| Peptide 6G | 0→ 80% buffer B | 28.5 | 9.4 / 33.0 |
| Peptide 6R | 0→ 80% buffer B | 31.7 | 20.4 / 64.3 |

The purified peptides obtained were further analysed by analytical RP-HPLC using a longer acetonitrile gradient and the molecular weights confirmed by mass spectroscopy. As can be seen in Table IV, peptides 1, 2, 3G, 4, 5 and 6G all have spectra corresponding to the calculated mass. The peptides 3R and 6R were found to contain a deletion product, peptide 6R with the methionine missing and peptide 3R with an arginine deletion. In order to separate the deletion product from the full-length peptide, optimisation of the elution gradient is required whereby the gradient length is increased considerably.

### Electrospray Ionisation Mass Spectroscopy

The molecular weights (MW) of the peptides were determined by Electrospray lonisation Mass Spectroscopy (ESI-MS) using a Micromass platform (II) quadrupole MS with Electrospray source and Masslynx NT version 3.2 software (Micromass, Cheshire, UK). The peptides were dissolved in a 1.1 mixture of 50% (v/v) acetonitrile/water and 3% (v/v) formic acid. The scan range was 0-2000 m/z, and samples were injected via a manual injector at a rate of 10 µl/min.

**Table IV. Calculated and observed molecular weight of peptides**

| **Peptide** | **Calculated MW** | **Observed MW** |
|---|---|---|
| Peptide 1 | 1502.57 | 1501.6 (M-1) |
| Peptide 2 | 1654.72 | 1653.5 (M-1) |
| Peptide 3G | 1367.45 | 1366.52 (M-1) |
| Peptide 3R | 1466.58 | 1465.66 (M-1) 1309.66 (Arg miss.) |
| Peptide 4 | 621.62 | 622.2 (M+1) |
| Peptide 5 | 664.24 | 665.2 (M+1) |
| Peptide 6G | 622.65 | 623.6 (M+1) |
| Peptide 6R | 721.78 | 722.3 (M+1) 591.3 (Met miss.) |

### Expression of target protein (glutathione S-transferase, GST) with affinity tags

### Expression

The expression of GST-tag fusion proteins was carried out at different scales, whereby single colonies of *E. coli* BL 21 host cells containing the recombinant GST-tag plasmids (Tag 1 to 6, untagged GST and vector only) were inoculated directly into 10 ml of 2xYT medium (16g/l Tryptone, 10 g/l yeast extract, 5 g/l NaCl, containing 100µg/ml of Ampicillin). The cultures were incubated overnight at 37°C with vigorous shaking. 10 ml aliquots of each of these cultures were added to sterile 2-liter baffled flasks containing 500 ml of 2xYT medium and incubated at 37°C with shaking until the absorbance at 600 nm (A₆₀₀) reached 0.5-1.0. IPTG was added to a final concentration of 1 mM, and the incubation was continued for an additional 3 hours at 28°C. The cultures were transferred to centrifuge containers and centrifuged at 7700xg for 10 min at 4°C to sediment the cells. The supernatant was discarded, and the cell pellets were resuspended in lysate buffer containing 1x PBS, 5mM EDTA and 1mM PMSF. The solutions were adjusted to a total volume of 30 ml, and a hen-egg-white lysozyme solution added (50 mg/ml, 1/100 of total volume). Following incubation of the solutions on ice for 10 min, solutions of MgCl₂ (2M, 1/1000 of total volume) and DNAse-I (10mg/ml, 1/1000 of volume) were then added, and the solutions were incubated again for 20 min on ice. Finally, the suspended cells were disrupted by sonication on ice with three short 30 sec burst with a 30 sec pause between each sonication.

The lysate was separated from cell debris by centrifugation in a SS34 rotor at 13000xg for 20 min at 4°C. The supernatants were retained for purification.

### SDS PAGE analysis

Aliquots (32 µl) of each of the fractionated sample (SM) and the chromatographically eluted samples [flow-through (FT), wash (W2) and elution (E)] were transferred into tubes, and 5x Sample Buffer (8 µl) (*vide infra*) was added. These samples were heated for 90 seconds at 90 °C, and 25 µl of each was introduced into respective wells of a 15% SDS-polyacrylamide gel using a pipette. The SDS- polyacrylamide gels were 15% acrylamide gels (1 mm thick) with a 4 % Stacker, and were prepared as follows:

| | Resolving Gel | Stacker |
|---|---|---|
| Acrylamide/Bisacrylamide solution (30%/0.8%) | 10 ml | 0.88 ml |
| 1.5M Tris-Cl pH 8.8 | 5 ml | - |
| 0.5M Tris-Cl pH 6.8 | - | 1.66 ml |
| 10% SDS | 0.2 ml | 66 µl |
| H₂O | 4.7 ml | 4.0 ml |
| 10% APS | 100 µl | 33.4 µl |
| TEMED | 14 µl | 7 µl |

| Sample Buffer: | |
|---|---|
| The 5x Sample Buffer consists of: | |
| 1.5M Tris-Cl pH 8.8 | 1.5 ml |
| Glycerol | 2.5 ml |
| SDS | 0.5 g |
| Bromophenol blue | 2 mg |
| Betamercaptoethanol | 1.0 ml |
| Water | up to a final volume of 5.0 ml |

The SDS-polyacrylamide gels were run in Tank Running Buffer (0.025M Tris, 0.192M glycine, 0.1% SDS, pH 8.3) using the Hoeffer Mini VE Vertical Electrophoresis System at a constant current of 20 mA per gel, until the dye-front reached the bottom of the resolving gel.

### Staining

### Silver stain:

The gels were stained using the silver staining method of Swain and Ross¹⁷, as indicated below:

| Silver stain protocol (per gel) | | | |
|---|---|---|---|
| Step | Reagent | Volume | Time (min) |
| 1. Fix | 40% Ethanol | 80ml | 10 |
| | 10% Acetic acid | 20ml | |
| | Water | 100ml | |
| 2. Rinse | Water | 200ml | 10 |
| 3. Fix/sensitize | Glutaraldehyde | 200µl | 5 |
| | Formaldehyde | 54µl | |
| | Ethanol | 80ml | |
| | Water | 120ml | |
| 4. Rinse | 40% Ethanol | 200ml | 20 |
| 5. Rinse | Water | 200ml | 20 |
| 6. Sensitize | Sodium thiosulfate | 0.05g | 1 |
| | Water | 250ml | - |
| 7. Rinse | Water | 200ml | 1 |
| 8. Rinse | Water | 200ml | 1 |
| 9. Silver | Silver nitrate | 0.2g | 20 |
| | Water | 200ml | |
| 10. Rinse | Water | 200 ml | 1 |
| 11. Develop | Sodium carbonate | 10g | |
| | Formaldehyde | 160µl | |
| | Water | 400ml | |
| 12. Stop | 5 % acetic acid | | |

### Coomassie Blue stain:

The gels were stained overnight using Coomassie stain, which stains the entire gel blue. On the following day the gel is de-stained, whereby only the protein bands retain the blue colour.

| Stain | |
|---|---|
| Coomassie | 2.5 g |
| Methanol | 450ml |
| Water | 450ml |
| Acetic acid | 100ml |

| Rapid de-stain | |
|---|---|
| Methanol | 300ml |
| Water | 600ml |
| Acetic acid | 100ml |

### Results

The tags were introduced to the protein GST by recombinant DNA technology. GST was used as model protein because it is commercially available, easy to express, well characterised and purified easily from cell lysates using Glutathione Sepharose™ 4B. Expression of the tagged-GST protein molecules is achieved using host bacterial cells (Escherichia coli BL21). These cells were induced with IPTG to express the tagged-GST proteins. The successfully purified tagged GST proteins from the contaminating host cell proteins were analysed by SDS-Page. As can be seen in Scheme 9, below, the starting material (SM) and flow through (FT) contain a lot of contaminating host proteins of various sizes. The elution fraction from the purification (E) shows that for each tagged-GST, there is a band corresponding to the theoretical sizes (∼26kDa), and that the eluted protein is relatively pure. Tag1-GST, Tag2-GST, Tag4-GST and Tag6-GST have a smaller band below the main band that may correspond to truncated tagged-GST formed via the action of host cell proteases.

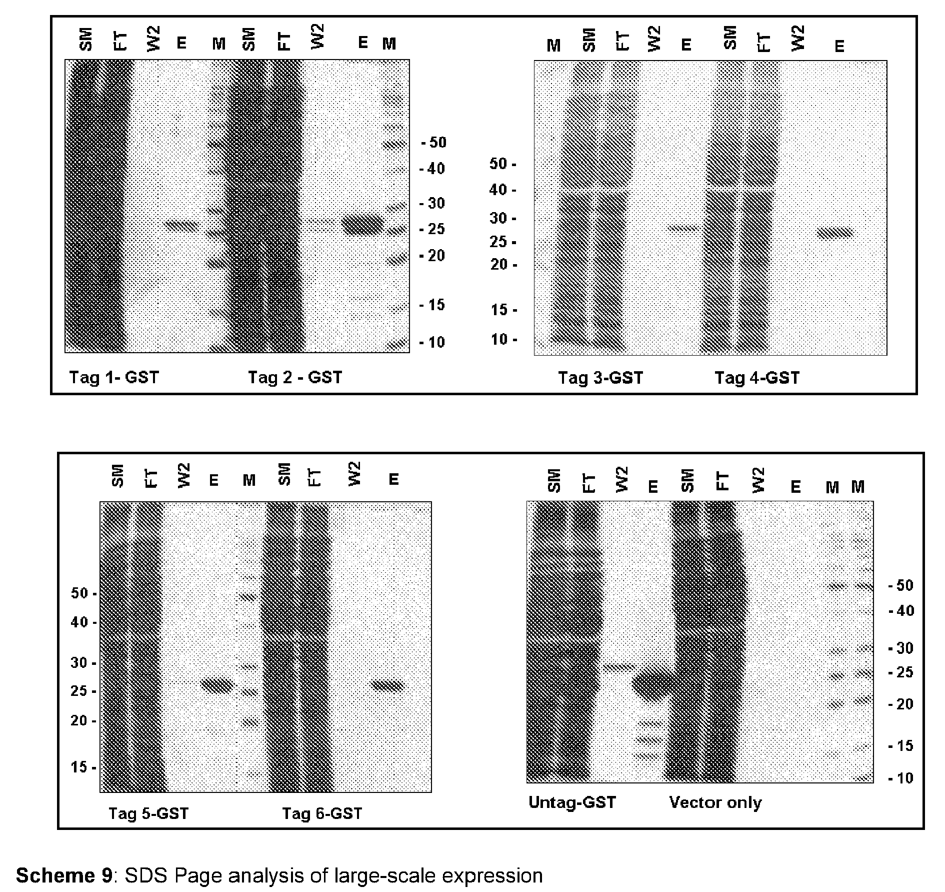
SM FT W2 E M SM FT W2 E M
- 50
- 40
- 30
- 25
- 20
- 15
- 10
Tag 1- GST Tag 2 - GST

M SM FT W2 E SM FT W2 E
50 -
40 -
30 -
25 -
20 -
15 -
10 -
Tag 3-GST Tag 4-GST

SM FT W2 E M SM FT W2 E
50 -
40 -
30 -
25 -
20 -
15 -
Tag 5-GST Tag 6-GST

SM FT W2 E SM FT W2 E M M
- 50
- 40
- 30
- 25
- 20
- 15
- 10
Untag-GST Vector only

Scheme 9: SDS Page analysis of large-scale expression

SDS-PAGE analysis of the affinity of the host proteins towards the IMAC gel using the vector confirmed that there are few/no low abundance host proteins that are able to be co-purified with the tagged-GST proteins. The purified protein was run on a single gel for comparison of the expressed sizes. As expected, tags 1-3 (long tags, see Scheme 9) are slightly larger than tags 4-6, and tags 2, 4 and 6 have some minor contaminant/truncation products (Scheme 10):

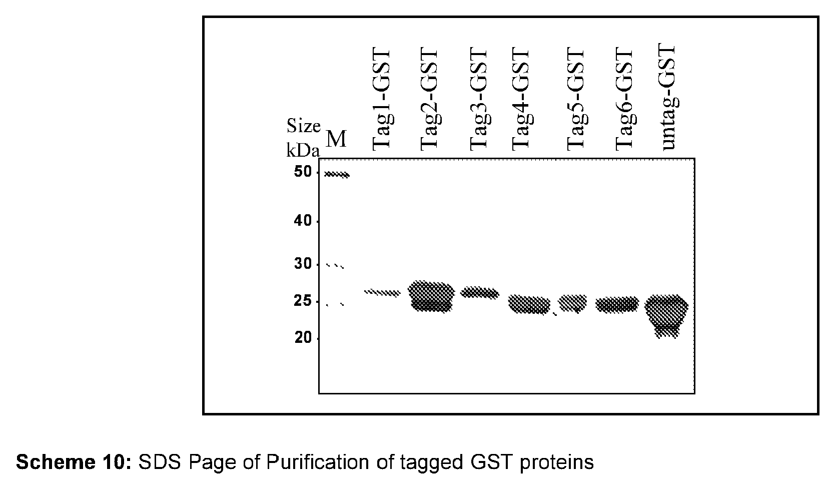
Size
kDa
M
Tag1-GST
Tag2-GST
Tag3-GST
Tag4-GST
Tag5-GST
Tag6-GST
untag-GST
50
40
30
25
20
Scheme 10: SDS Page of Purification of tagged GST proteins

Further characterisation of these tagged-GST proteins by MALDI-TOF mass spectroscopy show that all have expressed full length protein of expected molecular weight (Table V). The contaminants observed for tag 2-, 4- and 6-GST correspond to a full truncation of the tag from the rest of the GST protein. Given that these truncated products represent less than 10% of the total product, and will also be unable to bind to the *im-M*ⁿ⁺ gels (where is a 2+ or 3+ metal ion, e.g. Ca²⁺), these protein samples can still be used for subsequent analysis of Ca²⁺ (or other metal ion) binding.

**Table V: Tagged-GST mass spectroscopy results**

| Tagged-GST | Theor. MW (Da) | Observed MW (Da) | Standard Deviation |
|---|---|---|---|
| Tag 1 | 27298 | (1) 27312 (2) 25778 | 0.05 |
| Tag 2 | 27451 | (1) 27469 (2) 25827 | 0.06 |
| Tag 3 | 27163 | (1) 27176 | 0.04 |
| Tag 4 | 26418 | (1) 26410 (2) 25826 | 0.03 |
| Tag 5 | 26461 | (1) 26454 | 0.02 |
| Tag 6 | 26418 | (1) 26421 (2)25833 | 0.01 |
| untag | 25814 | (1) 25827 | 0.05 |

### List of References

(1) Porath, J. Prot. Expr. Purif., 1992, 3, 263-281.
(2) Kovacs, Z., Sherry, A.D.J. J.Chem.Soc., Chem.Commun., 1995, 185.
(3) Dischino, D. D.; Delaney, E. J.; Emswiler, J. E.; Gaughan, G. T.; Prasad, J. S.; Srivastava, S. K.; Tweedle, M. F. Inorg. Chem., 1991, 30, 1265-1269.
(4) Sun, X.; Wuest, M.; Kovacs, Z.; Sherry, A. D. J.Biol.Inorg. Chem., 2003, 8, 217-225.
(5) Burai, L., Ren, J., Kovacs, Z., Bruecher, E., Sherry, A.D. Inorg. Chem., 1998, 37, 69-75.
(6) Kovacs, Z., Sherry, A.D.J. Synthesis, 1997, 759.
(7) Richman, J. E., Atkins, T.A. J. Am. Chem. Soc., 1974, 96, 2268-2270.
(8) Parker, D. Macrocycle Synthesis, a practical approach; Oxford University Press: New York, 1996; Vol. 2.
(9) Atkins, T. J. U.S., 1978.
(10) Weisman, G. R.; Vachon, D. J.; Johnson, V. B.; Gronbeck, D. A. J.Chem.Soc., Chem.Commun., 1987, 886.
(11) Zachariou, M. PhD Thesis, 1994, Department of Biochemistry, Monash University.
(12) Porath, J., Olin, B. Biochemistry, 1983, 22, 1621-1630.
(13) Jennings, M. L. Annu. Rev. Biochem., 1989, 58, 999-1027.
(14) Babu Nature, 1985, 315, 37-40.
(15) Novabiochem Novabiochem catalog and peptide synthesis handbook, 1999.
(16) Hearn, M. T. W., Acosta, D. J. Mol. Recognit., 2001, 14, 323-369.
(17) Swain, M.; Ross, N. W. Electrophoresis, 1995, 16, 948-951.
(18) Chaga, G., Andersson, B. and Porath, J. J. Chromatogr. A, 1996, 732, 261-269.
(19) Richman, J. E. and Atkins, T. J., J. Am. Chem. Soc., 1974, 96, 2268.
(20) Parker, D. Macrocycle Synthesis, a Practical Approach. Oxford: Oxford University Press; 1996.

## Claims

1. A method for purifying a protein of interest, the method comprising the steps of:
contacting a protein sample containing a polypeptide, comprising said protein of interest together with other proteins,
wherein said polypeptide is an oligopeptide comprising an amino acid sequence selected from the group consisting of :
| | |
|---|---|
| MDADGNGTIDFAEF | (SEQ. ID No. 1), |
| MDIDGDGHINYEEF | (SEQ. ID No. 2), |
| MDVDGSGTIGSSEL | (SEQ. ID No. 3), |
| MDVDRSGTIGSSEL | (SEQ. ID No. 4), |
| MDADGN | (SEQ. ID No. 5), |
| MDIDGD | (SEQ. ID No. 6), |
| MDVDGS | (SEQ. ID No. 7), |
| MDVDRS | (SEQ. ID No. 8), |
and further consisting of: variants of said sequences wherein one or more amino acid residues in a sequence chosen among said sequences are replaced with an amino acid residue of similar chemical functionality to the the replaced amino acid residue,
with a metal ion-containing functionalized polymer substrate,
wherein said polymer substrate is a polymer substrate functionalized with a functionality comprising at least one cyclic, metal ion coordinating ligand group which comprises at least 3 nitrogen donor atoms in the ring of said cyclic group, at least one of said nitrogen atoms having an optionally substituted carboxy(lower alkyl) or optionally substituted phosphono(lower alkyl) group covalently attached thereto, and wherein said functionalized polymer substrate further comprises a metal ion coordinated to at least one of said cyclic ligand groups in said functionality,
under conditions whereby said polypeptide binds to said metal ion-containing functionalized polymer substrate so as to form a complex therewith;
washing the complex with a buffer solution to remove said other proteins; and
eluting the bound polypeptide from the washed complex.

2. A method according to any one of the preceding claims, wherein said cyclic, metal ion coordinating ligand group is derived from a heterocycle chosen among:
triazacycloalkanes and -cycloalkenes; and
tetraazacycloalkanes and -cycloalkenes.

3. A method according to any one of the preceding claims, wherein said cyclic, metal ion coordinating ligand group is chosen among
a. triazacycloalkanes and -cycloalkenes, wherein at least two of the three ring N atoms have an optionally substituted carboxy(lower alkyl) or optionally substituted phosphono(lower alkyl) group covalently attached thereto; and
b. tetraazacycloalkanes and -cycloalkenes, wherein at least two of the four ring N atoms have an optionally substituted carboxy(lower alkyl) or optionally substituted phosphono(lower alkyl) group covalently attached thereto.

4. A method according to any one of the preceding claims, wherein said cyclic, metal ion coordinating ligand group is derived from a heterocycle chosen among:
1,4,7-triazacyclononane;
1,4,7-triazacyclodecane;
1,4,8-triazacycloundecane;
1,5,9-triazacyclododecane;
1,4,7,10-tetraazacyclododecane;
1,4,7,10-tetraazacyclotridecane;
1,4,7,11-tetraazacyclotetradecane;
1,4,8,11- tetraazacyclotetradecane;
1,4,8,12-tetraazacyclopentadecane; and
1,5,9,13-tetraazacyclohexadecane.

5. A method according to any one of the preceding claims, wherein said cyclic metal ion coordinating ligand group is selected from the group consisting of:
1,4-bis(carboxymethyl)-1,4,7-triazacyclononane (T2A),
1,4-bis(phosphonomethyl)-1,4,7-triazacyclononane (T2P),
1,7-bis-(phosphonomethyl)-1,4,7,10-tetraazacyclododecane (D02P),
1,4,7-tris(carboxymethyl)-1,4,7,10-tetraazacyclododecane (D03A), and
1,4,7-tris(phosphonomethyl)-1,4,7,10-tetraazacyclododecane (D03P).

6. A method according to any one of the preceding claims, further comprising a step wherein said oligopeptide is cleaved from said polypeptide.

7. A method according to any one of the preceding claims wherein said oligopeptide is cleaved from said polypeptide by enzymatic means, such as by use of an endopeptidase or exopeptidase.

8. The method according to any one of the preceding claims, wherein said coordinated metal ion is a divalent or trivalent metal ion, such as a metal ion selected from the group consisting of Ca²⁺, Mg²⁺, Zn²⁺ and Fe³⁺.

9. A polymer substrate functionalized with a functionality comprising at least one cyclic, metal ion coordinating ligand group which comprises at least 3 nitrogen donor atoms in the ring of said cyclic group, at least one of said nitrogen atoms having an optionally substituted carboxy(lower alkyl) or optionally substituted phosphono(lower alkyl) group covalently attached thereto,
wherein when said cyclic, metal ion coordinating ligand group is derived from the heterocycle triazacycloalkanes or -cycloalkenes then at least two of the three ring N atoms have an optionally substituted carboxy(lower alkyl) or optionally substituted phosphono(lower alkyl) group covalently attached thereto.

10. A functionalized polymer according to any one of the preceding claims, wherein cyclic metal ion coordinating ligand group is selected from the group consisting of:
1,4-bis(carboxymethyl)-1,4,7-triazacyclononane (T2A),
1,4-bis(phosphonomethyl)-1,4,7-triazacyclononane (T2P),
1,7-bis-(phosphonomethyl)-1,4,7,10-tetraazacyclododecane (D02P),
1,4,7-tris(carboxymethyl)-1,4,7,10-tetraazacyclododecane (D03A), and
1,4,7-tris(phosphonomethyl)-1,4,7,10-tetraazacyclododecane (D03P).

11. A functionalized polymer substrate according to claim 9, wherein said cyclic, metal ion coordinating ligand group is derived from a heterocycle chosen among:
1,4,7-triazacyclononane;
1,4,7-triazacyclodecane;
1,4,8-triazacycloundecane;
1,5,9-triazacyclododecane;
1,4,7,10-tetraazacyclododecane;
1,4,7,10-tetraazacyclotridecane;
1,4,7,11-tetraazacyclotetradecane;
1,4,8,11- tetraazacyclotetradecane;
1,4,8,12-tetraazacyclopentadecane; and
1,5,9,13-tetraazacyclohexadecane.

12. A functionalized polymer substrate according to any one of the preceding claims, further comprising a metal ion coordinated to at least one of said cyclic ligand groups in said functionality, wherein said coordinated metal ion is optionally a divalent or trivalent metal ion, such as a metal ion selected from the group consisting of Ca²⁺, Mg²⁺, Zn²⁺ and Fe³⁺.

13. A functionalized polymer substrate according to any one of the preceding claims, wherein said polymer is substantially water-insoluble.

14. A process for preparing a functionalized polymer substrate according to any one of the preceding claims, comprising the steps of:
selecting a polymer substrate having a reactive functional group capable of undergoing a first reaction with a first functional group of a bifunctional reagent having a first and a second functional group,
said first reaction resulting in covalent bond formation between said polymer substrate and said bifunctional reagent,
said second functional group of the resulting covalently bound reagent being subsequently capable of undergoing a second reaction with a reactive ring -NH-group present in a species comprising at least one cyclic, metal ion coordinating ligand group which comprises at least 3 nitrogen donor atoms in the ring of said cyclic group, at least one of said nitrogen atoms having an optionally substituted carboxy(lower alkyl) or optionally substituted phosphono(lower alkyl) group covalently attached thereto,
said second reaction resulting in covalent bond formation between said species and said covalently bound reagent;
reacting said polymer substrate with said bifunctional reagent; and
reacting said resulting covalently bound reagent with said species.

15. A process for preparing a functionalized polymer substrate according to any one of claims 9-13 and further comprising a metal ion coordinated to at least one of said cyclic groups, the process comprising contacting a functionalized polymer substrate according to any one of claims 9-13 with an aqueous solution of an inorganic or organic salt of said metal ion.
